(19) Europäisches Patentamt
European Patent Office
Office européen des brevets

(11) **EP 1 944 733 B1**

(12) **EUROPEAN PATENT SPECIFICATION**

(45) Date of publication and mention
of the grant of the patent:
**25.12.2019 Bulletin 2019/52**

(51) Int Cl.:
***G06T 5/50*** *(2006.01)*   ***G06T 7/00*** *(2017.01)*
***A61B 5/00*** *(2006.01)*

(21) Application number: **08100161.2**

(22) Date of filing: **07.01.2008**

(54) **System and method for superimposing a representation of the tip of a catheter on an image acquired by a moving imager**

Anordnung und Verfahren zur Überlagerung einer Darstellung der Spitze eines Katheters auf einem von einem mobilen Bildgeber erfassten Bild

Système et procédé de superposition d'une représentation de la pointe d'un cathéter sur une image acquise par un imageur en mouvement

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **10.01.2007 US 879672 P**

(43) Date of publication of application:
**16.07.2008 Bulletin 2008/29**

(73) Proprietor: **St. Jude Medical International Holding S.à r.l.**
**2449 Luxembourg (LU)**

(72) Inventors:
• **Strommer, Gera**
**34759, HAIFA (IL)**
• **Eichler, Uzi**
**34657, HAIFA (IL)**
• **Schwartz, Liat**
**34349, HAIFA (IL)**
• **Shmarak, Itzik**
**36001, NOFIT (IL)**
• **Peles, David**
**34735 Haifa (IL)**

(74) Representative: **Kramer Barske Schmidtchen Patentanwälte PartG mbB**
**European Patent Attorneys**
**Landsberger Strasse 300**
**80687 München (DE)**

(56) References cited:
**US-A1- 2005 033 149    US-A1- 2006 058 647**

• **GORGES S ET AL: "Model of a Vascular C-Arm for 3D Augmented Fluoroscopy in Interventional Radiology", MEDICAL IMAGE COMPUTING AND COMPUTER-ASSISTED INTERVENTION - MICCAI 2005 LECTURE NOTES IN COMPUTER SCIENCE;;LNCS, SPRINGER, BERLIN, DE, vol. 3750, 1 January 2005 (2005-01-01), pages 214-222, XP019021757, ISBN: 978-3-540-29326-2**

**Description**

**FIELD OF THE DISCLOSED TECHNIQUE**

The disclosed technique relates to medical navigation systems in general, and to methods for combining medical imaging systems with medical navigation systems, in particular.

**BACKGROUND OF THE DISCLOSED TECHNIQUE**

US 2006/058647 A1 relates to medical operations in general and to methods and systems for mounting a stent in a body of a patient, in particular.

Catheters are employed for performing medical operations on a lumen of the body of a patient, such as percutaneous transluminal coronary angioplasty (PTCA), percutaneous transluminal angioplasty (PTA), vascularizing the lumen, severing a portion of the lumen or a plaque there within (e.g., atherectomy), providing a suture to the lumen, increasing the inner diameter of the lumen (e.g., by a balloon, a self expanding stent, a stent made of a shape memory alloy (SMA), or a balloon expanding stent) and maintaining the increased diameter by implanting a stent. During these medical operations, it is advantageous for the physical staff to view an image of the tip of the catheter or a representation thereof, against a real-time image of a portion of the body of the patient. Such devices are known in the art.

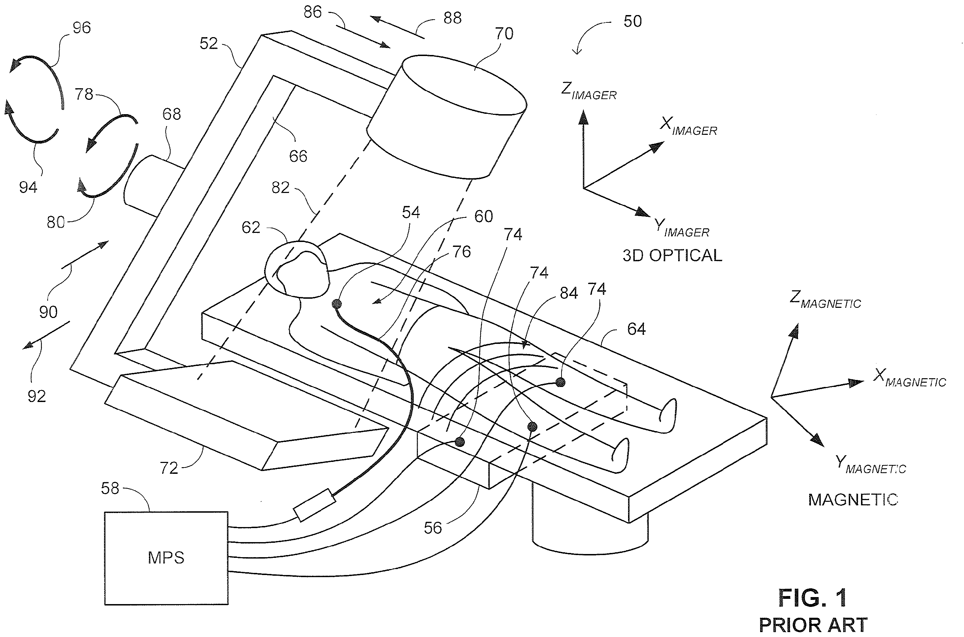

Reference is now made to Figure 1, which is a schematic illustration of a system, generally referenced 50, for determining the position of the tip of a catheter relative to images of the body of a patient detected by a moving imager, as known in the art. System 50 includes a moving imager 52, a positioning sensor 54, a transmitter assembly 56 and a magnetic positioning system 58. Moving imager 52 is a device which acquires an image (not shown) of a body region of interest 60 of the body of a patient 62 lying on an operation table 64.

Moving imager 52 includes a moving assembly 66, a moving mechanism 68, an intensifier 70 and a emitter 72. Transmitter assembly 56 includes a plurality of magnetic field generators 74. In the example set forth in Figure 1, moving imager 52 is an X-ray type imager (known in the art as C-arm imager). Hence, intensifier 70 and emitter 72 are connected with moving assembly 66, such that intensifier 70 is located at one side of patient 62 and emitter 72 is located at an opposite side of patient 62. Intensifier 70 and emitter 72 are located on a radiation axis (not shown), wherein the radiation axis crosses the body region of interest 60.

Transmitter assembly 56 is fixed below operation table 64. positioning sensor 54 is located at a distal portion (not shown) of a catheter 76. Catheter 76 is inserted to the body region of interest 60. positioning sensor 54 and magnetic field generators 74 are connected with magnetic positioning system 58. Moving imager 52 is associated with an $X_{IMAGER}$, $Y_{IMAGER}$, $Z_{IMAGER}$ coordinate system (i.e., 3D optical coordinate system). Magnetic positioning system 58 is associated with an $X_{MAGNETIC}$, $Y_{MAGNETIC}$, $Z_{MAGNETIC}$ coordinate system (i.e., magnetic coordinate system). The 3D optical coordinate system and the magnetic coordinate system are different (i.e., the scales, origins and orientations thereof are different). Moving mechanism 68 is connected to moving assembly 66, thereby enabling moving assembly 66 to rotate about the $Y_i$ axis. Moving mechanism 68 rotates moving assembly 66 in directions designated by arrows 78 and 80, thereby changing the orientation of the radiation axis on the $X_{IMAGER}$-$Z_{IMAGER}$ plane and about the $Y_{IMAGER}$ axis.

Moving mechanism 68 rotates moving assembly 66 in directions designated by arrows 94 and 96, thereby changing the orientation of the radiation axis on the $Z_{IMAGER}$-$Y_{IMAGER}$ plane and about the $X_{IMAGER}$ axis. Moving imager 52 can include another moving mechanism (not shown) to move moving imager 52 along the $Y_{IMAGER}$ axis in directions designated by arrows 86 and 88 (i.e., the cranio-caudal axis of patient 62). Moving imager 52 can include a further moving mechanism (not shown) to move moving imager 52 along the $X_{IMAGER}$ axis in directions designated by arrows 90 and 92 (i.e., perpendicular to the cranio-caudal axis of patient 62).

Emitter 72 emits radiation at a field of view 82 toward the body region of interest 60, to be detected by intensifier 70, thereby radiating a visual region of interest (not shown) of the body of patient 62. Intensifier 70 detects the radiation which is emitted by emitter 72 and which passes through the body region of interest 60. Intensifier 70 produces a two-dimensional image (not shown) of body region of interest 60, by projecting a three-dimensional image (not shown) of body region of interest 60 in the 3D optical coordinate system, on a 2D optical coordinate system (not shown) respective of intensifier 70. A display (not shown) displays this two-dimensional image in the 2D optical coordinate system.

Magnetic field generators 74 produce a magnetic field 84 in a magnetic region of interest (not shown) of the body of patient 62. Magnetic positioning system 58 determines the position of the distal portion of catheter 76 in the magnetic coordinate system, according to an output of positioning sensor 54. The display displays a representation of the distal portion of catheter 76 against the two-dimensional image of the body region of interest 60, according to an output of magnetic positioning system 58.

Since the 3D optical coordinate system and the magnetic coordinate system are different, the data produced by intensifier 70 and by magnetic positioning system 58 are transformed to a common coordinate system (i.e., to the magnetic coordinate system), according to a transformation matrix, before displaying the representation of the distal portion of catheter 76 against the two-dimensional image of the body region of interest 60. Transmitter assembly 56 is fixed to a predetermined location underneath operation table 64. As moving imager 52 moves relative to the body of patient 62, there are instances at which the magnetic region of interest does not coincide with the visual field of interest.

US Patent No. 6,203,493 B1 issued to Ben-Haim and entitled "Attachment With One or More Sensors for Precise Position Determination of Endoscopes" is directed to a plurality of sensors for determining the position of any point along a colonoscope. The colonoscope includes a flexible endoscopic sheath, an endoscopic insertion tube, and a control unit. The endoscopic insertion tube passes through a lumen within the flexible endoscopic sheath. The flexible endoscopic sheath includes a plurality of work channels.

The endoscopic insertion tube is a non-disposable elongate tube which includes electrical conducting materials. Each of the sensors measures at least three coordinates. The sensors are fixed to the endoscopic insertion tube and connected to a position determining system. The flexible endoscopic sheath is an elongate disposable tube which includes materials which do not interfere with the operation of the position determining system. In this manner, the position determining system can determine the position of any point along the flexible endoscopic sheath and the endoscopic insertion tube.

US Patent No. 6,366,799 B1 issued to Acker et al., and entitled "Movable Transmit or Receive Coils for Location System", is directed to a system for determining the disposition of a probe inserted into the body of a patient. The probe includes one or more field transducers. The system includes a frame, a plurality of reference field transducers and a drive circuitry. The reference field transducers are fixed to the frame, and the frame is fixed to an operating table beneath a thorax of the patient which is lying on the operating table. The reference field transducers are driven by the drive circuitry. The field transducers of the probe generate signals in response to magnetic fields generated by the reference field transducers, which allows determining the disposition of the probe.

In another embodiment, the patent describes a movable transducer assembly which includes a flexible goose neck arm, a plurality of reference transducers, a support, and an adjustable mounting mechanism. The reference transducers are fixed to the support. The flexible goose neck arm is fixed to the support and to the adjustable mounting mechanism. The adjustable mounting mechanism is mounted to an operating table. The flexible goose neck allows a surgeon to move the support and the reference transducers to a position close to the region of interest during the surgical procedure and to reposition away from areas to which the surgeon must gain access to.

Methods for correcting distortions in an image acquired by a C-arm imager are known in the art. One such method employs a grid located in front of the intensifier. The real shape of this grid is stored in a memory. The acquired image includes an image of the grid. In case the acquired image is distorted, the shape of the grid on the acquired image is also distorted. An image processor detects the distortion of the grid in the acquired image, and corrects for the distortion according to the real shape of the grid stored in the memory.

## SUMMARY OF THE DISCLOSED INVENTION

It is an object of the disclosed invention to provide a novel method and system according to the independent claims, for superimposing a representation of the tip of a catheter on an image of the body of a patient.

In accordance with the disclosed invention, there is thus provided a method for displaying a representation of the tip of a medical device located within a body region of interest of the body of a patient, on an image of the body region of interest, the image being acquired by an image detector of a moving imager. The method includes the procedures of acquiring a medical positioning system (MPS) sensor image of an MPS sensor, determining a set of intrinsic and extrinsic parameters, and determining two-dimensional optical coordinates of the tip of the medical device. The method further includes the procedures of superimposing the representation of the tip of the medical device, on the image of the body region of interest, and displaying the representation of the tip of the medical device superimposed on the image of the body region of interest.

The MPS sensor image of the MPS sensor is acquired by the image detector, at a physical zoom setting of the image detector respective of the image, and at a selected image detector region of interest setting of the image detector. The MPS sensor is associated with an MPS. The MPS sensor responds to an electromagnetic field generated by an electromagnetic field generator, firmly coupled with a moving portion of the moving imager.

The set of intrinsic and extrinsic parameters is determined according to sensor image coordinates of the MPS sensor image, in a two-dimensional optical coordinate system respective of the image detector, and according to non-real-time MPS coordinates of the MPS sensor, in an MPS coordinate system respective of the MPS. The two-dimensional optical coordinates of the tip of the medical device, are determined according to the physical zoom setting, according to the set of intrinsic and extrinsic parameters, according to the selected image detector region of interest setting, and according to real-time MPS coordinates of an MPS sensor located at the tip of the medical device. The representation of the tip of the medical device is superimposed on the image of the body region of interest, according to the two-dimensional optical coordinates. Determining said set of intrinsic and extrinsic parameters is performed with respect to a plurality of physical zoom settings of said image detector, by interpolating between two adjacent ones of said physical zoom settings, or by extrapolating beyond two adjacent ones of said physical zoom settings.

In accordance with another aspect of the disclosed invention, there is thus provided a system for displaying a representation of the tip of a medical device located within a body region of interest of a patient, on an image of the body region of interest, the image being acquired by an image detector of a moving imager. The system includes a magnetic field generator, a medical device medical positioning system (MPS) sensor, an MPS, and a processor. The magnetic field generator is firmly coupled with a moving portion of the moving imager. The medical device MPS sensor is coupled with the tip of the medical device. The MPS is coupled with the magnetic field generator and with the medical device MPS sensor. The processor is coupled with the MPS.

The magnetic field generator produces a magnetic field at the body region of interest. The medical device MPS sensor detects the magnetic field. The magnetic field generator is associated with an MPS coordinate system respective of the MPS. The MPS determines the MPS coordinates of the medical device MPS sensor, according to an output of the medical device MPS sensor. The processor determines the two-dimensional coordinates of the tip of the medical device located within the body region of interest, according to a physical zoom setting of the image detector respective of the image, and according to a set of intrinsic and extrinsic parameters respective of the image detector. The processor determines the two-dimensional coordinates of the tip of the medical device, furthermore according to a selected image detector region of interest setting of the image detector, and according to the MPS coordinates of the medical device MPS sensor. The processor superimposes a representation of the tip of the medical device, on the image, according to the two-dimensional coordinates. Determining said set of intrinsic and extrinsic parameters is performed with respect to a plurality of physical zoom settings of said image detector, by interpolating between two adjacent ones of said physical zoom settings, or by extrapolating beyond two adjacent ones of said physical zoom settings.

**BRIEF DESCRIPTION OF THE DRAWINGS**

The disclosed technique will be understood and appreciated more fully from the following detailed description taken in conjunction with the drawings in which:

Figure 1 is a schematic illustration of a system or determining the position of the tip of a catheter, relative to images of the body of a patient detected by a moving imager, as known in the art;

Figure 2 is a schematic illustration of a system for displaying a representation of the tip of a medical device on the tip of a medical device, on a real-time image of the body of a patient, acquired by a moving imager, the position being determined according to the characteristics of the real-time image and those of the moving imager, the system being constructed and operative in accordance with an embodiment of the disclosed technique;

Figure 3 is a schematic illustration of a method for superimposing a representation of the tip of a medical device located within a body region of interest of the patient of

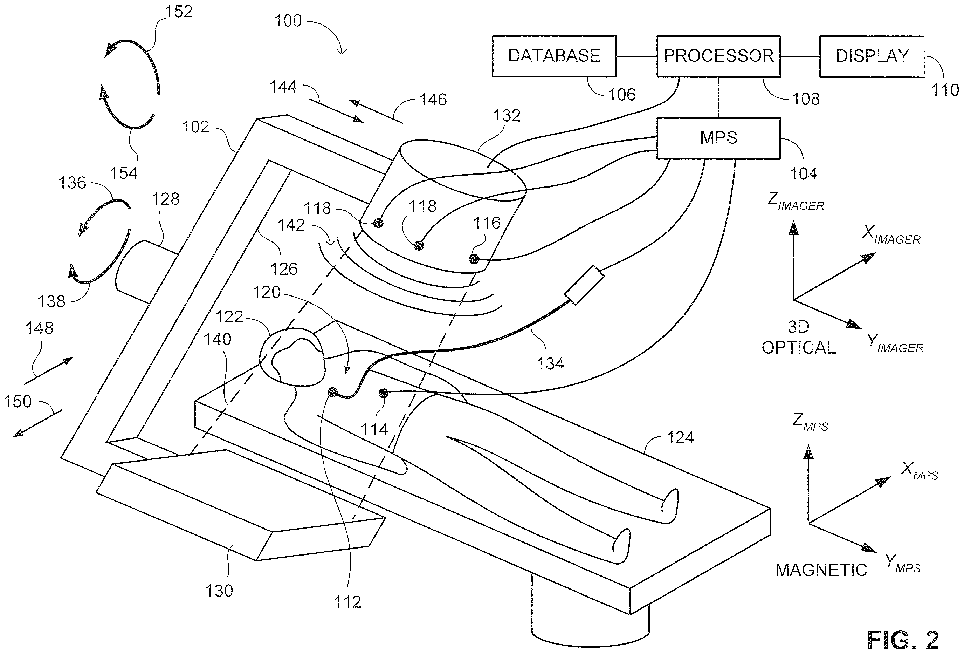

Figure 2, on an image of the body region of interest, acquired by the image detector of the system of Figure 2, operative according to another embodiment of the disclosed technique;

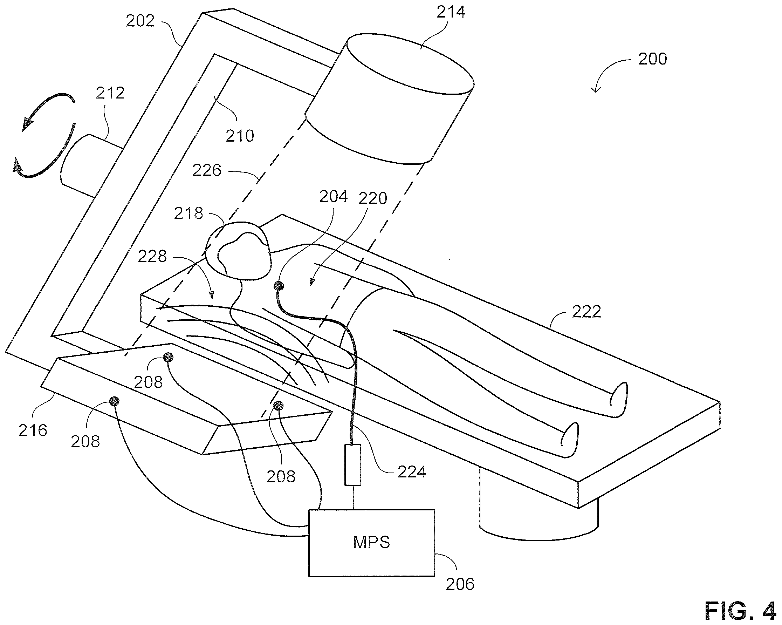

Figure 4 is a schematic illustration of a system for determining the position of the tip of a medical device, relative to images of the body of a patient detected by a moving imager, the system being constructed and operative in accordance with a further embodiment of the disclosed technique; and

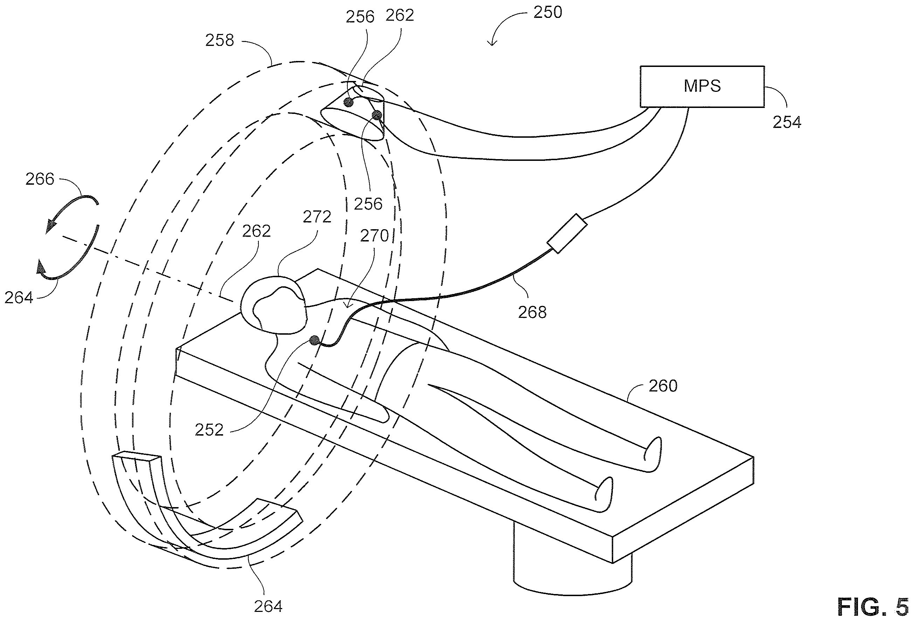

Figure 5 is a schematic illustration of a system for determining the position of the tip of a medical device relative to images of the body of a patient detected by a computer assisted tomography (CAT) machine, the system being constructed and operative in accordance with another embodiment of the disclosed technique.

**DETAILED DESCRIPTION OF THE EMBODIMENTS**

The disclosed technique overcomes the disadvantages of the prior art by determining a distortion correction model beforehand, corresponding to distortions which an image may undergo in real-time, and modifying the position of the projection of the tip of a catheter on the distorted real-time image, according to the distortion correction model. In this manner, a system according to the disclosed technique, can determine a substantially accurate position of the projection of the tip of the catheter on the distorted real-time image of the body of a patient, by retrieving data from a look-up table, without requiring any time consuming image processing in real time. Furthermore, as a result of firmly attaching the magnetic field generators of a medical positioning system (MPS) to an image detector of a moving imager, the origin of the 3D optical coordinate system of the image detector can be arbitrarily set at the origin of the magnetic coordinate system of the MPS, thereby reducing the processing load even further.

The term "cranio-caudal" axis herein below, refers to a longitudinal axis between the head of the patient and the toes of the patient. The term "medical device" herein below, refers to a vessel expansion unit such as a balloon catheter, stent carrying catheter, medical substance dispensing catheter, suturing catheter, guidewire, an ablation unit such as laser, cryogenic fluid unit, electric impulse unit, cutting balloon, rotational atherectomy unit (i.e., rotablator), directional atherectomy unit, transluminal extraction unit, drug delivery catheter, brachytherapy unit, intravascular ultrasound catheter, lead of a cardiac rhythm treatment (CRT) device, lead of an intra-body cardiac defibrillator (ICD) device, guiding device of a lead of a cardiac rhythm treatment device, guiding device of a lead of an intra-body cardiac defibrillator device, valve treatment catheter, valve implantation catheter, intra-body ultrasound catheter, intra-body computer tomography catheter, therapeutic needle, diagnostic needle, gastroenterology device (e.g., laparoscope, endoscope, colonoscope), orthopedic device, neurosurgical device, intra-vascular flow measurement device, intra-vascular pressure measurement device, intra-vascular optical coherence tomography device, intra-vascular near infrared spectroscopy device, intra-vascular infrared device (i.e., thermosensor), otorhinolaryngology precision surgery device, and the like.

The term "position" of an object herein below, refers to either the location or the orientation of the object, or both the location and orientation thereof. The term "magnetic region of interest" herein below, refers to a region of the body of the patient which has to be magnetically radiated by a magnetic field generator, in order for an MPS sensor to respond to the radiated magnetic field, and enable the MPS to determine the position of the tip of a medical device.

The term "image detector" herein below, refers to a device which produces an image of the visual region of interest. The image detector can be an image intensifier, flat detector (e.g., complementary metal-oxide semiconductor - CMOS), and the like. The term "magnetic coordinate system" herein below, refers to a three-dimensional coordinate system associated with the MPS. The term "3D optical coordinate system" herein below, refers to a three-dimensional coordinate system associated with a three-dimensional object which is viewed by the image detector. The term "2D optical coordinate system" herein below, refers to a two-dimensional coordinate system associated with the image detected by the image detector viewing the three-dimensional object.

The term "body region of interest" herein below, refers to a region of the body of a patient on which a therapeutic operation is to be performed. The term "visual region of interest" herein below, refers to a region of the body of the patient which is to be imaged by the moving imager. The term "image detector region of interest (ROI)" herein below, refers to different sizes of the detection region of the image detector. The image detector can detect the visual region of interest, either by utilizing the entire area of the image detector, or smaller areas thereof around the center of the image detector. The term "image detector ROI" refers to both an image intensifier and a flat detector.

The term "image rotation" herein below, refers to rotation of an image acquired by the image detector, performed by an image processor. The term "image flip" herein below, refers to a mirror image of the acquired image performed about an axis on a plane of the acquired image, wherein this axis represents the rotation of the acquired image about another axis perpendicular to the plane of the acquired image, relative to a reference angle (i.e., after performing the image rotation). For example, if the acquired image is rotated 25 degrees clockwise and an axis defines this amount of rotation, then the image flip defines another image obtained by rotating the acquired image by 180 degrees about this axis. In case no image rotation is performed, an image flip is performed about a predetermined axis (e.g., a substantially vertical axis located on the plane of the acquired image).

The term "intrinsic parameters" herein below, refers to optical characteristics of the image detector and an optical assembly of the moving imager, such as focal point, focal length, inherent optical distortion characteristics, and the like. In case of a moving imager in which the magnetic field generators are firmly attached to the periphery of the image detector, the ideal condition is for the visual region of interest and the magnetic region of interest to be identical. However, due to various constraints, this condition might not be fully satisfied. Therefore, it is necessary to determine a transformation matrix which defines the rotation and translation between the visual region of interest and the magnetic region of interest. The parameters of this transformation matrix are herein below referred to as "extrinsic parameters". The term "moving image detector" herein below, refers to an image detector in which the image detector moves linearly along an axis substantially normal to the surface of the emitter, and relative to the emitter, in order to zoom-in and zoom-out.

The term "reference image" herein below, refers to an image acquired by the image detector at calibration (i.e., off-line), when the moving imager is positioned at a selected reference position (e.g., 0, 0, 0 coordinates in the 3D coordinate system of the moving imager). The term "reference image distortion" herein below, refers to the distortion in the reference image. The term "viewing position image distortion" herein below, refers to the distortion in the image acquired by the image detector, at a selected position of the moving imager (e.g., the selected position). The viewing position image distortion is generally caused by the influence of the magnetic field of the Earth on the image intensifier. Thus, the image acquired by the image detector is distorted differently at different positions of the moving imager.

Generally, an image intensifier introduces significant viewing position distortions in an image acquired thereby, whereas a flat detector introduces substantially no distortion in the image. Therefore, the procedures for superimposing a representation of the tip of catheter on a real-time image of the body region of interest, according to the disclosed technique, are different in case of an image intensifier and a flat detector.

The term "image rotation distortion" herein below, refers to the image distortion due to image rotation. The term "image flip distortion" herein below, refers to the image distortion due to image flip. The image rotation distortion, image flip distortion, and viewing position image distortion in an image acquired by a flat detector, is negligible compared to those acquired by an image intensifier. It is noted that the image rotation distortion and the image flip distortion is substantially greater than the viewing position image distortion. The term "reference distortion correction model" herein below, refers to a transformation matrix which corrects the reference image distortion, when applied to the reference image.

The terms "off-line" and "non-real-time" employed herein below interchangeably, refer to an operating mode of the system, prior to the medical operation on the patient, such as calibration of the system, acquisition of pre-operational images by the image detector, determination of the intrinsic and extrinsic parameters, determination of the image rotation and image flip distortions associated with an image acquired by the image detector, entering data into a database associated with the system, and the like. The terms "on-line" and "real-time" employed herein below interchangeably, refer to an operating mode of the system during the medical operation on the patient.

Reference is now made to Figure 2, which is a schematic illustration of a system, generally referenced 100, for displaying a representation of the tip of a medical device on the tip of a medical device on a real-time image of the body of a patient, acquired by a moving imager, the position being determined according to the characteristics of the real-time image and those of the moving imager, the system being constructed and operative in accordance with an embodiment of the disclosed technique. System 100 includes a moving imager 102, a medical positioning system (MPS) 104, a database 106, a processor 108, a display 110, MPS sensors 112, 114 and 116, a plurality of magnetic field generators 118 (i.e., transmitters).

Moving imager 102 is a device which acquires an image (not shown) of a body region of interest 120 of the body of a patient 122 lying on an operation table 124. Moving imager 102 includes a moving assembly 126, a moving mechanism 128, an emitter 130, and an image detector 132.

Moving imager 102 can operate based on X-rays, nuclear magnetic resonance, elementary particle emission, thermography, and the like. Moving imager 102 has at least one degree of freedom. In the example set forth in Figure 2, moving imager 102 is a C-arm imager). Emitter 130 and image detector 132 are coupled with moving assembly 126, such that emitter 130 is located at one side of patient 122 and image detector 132 is located at the opposite side of patient 122. Emitter 130 and image detector 132 are located on a radiation axis (not shown), wherein the radiation axis crosses the body region of interest 120.

The system can further include a user interface (e.g., a push button, joystick, foot pedal) coupled with the moving imager, to enable the physical staff to sequentially rotate the image acquired by the image detector, to flip the image at a given rotation angle, or set the ROI of the image detector. The moving imager is constructed such that the image indexes forward or backward by a predetermined amount, at every activation of the push button. This index can be for example, five degrees, thus enabling the moving imager to perform a maximum of seventy two image rotations (i.e., 360 divided by 5). Since the moving imager can produce one image flip for each image rotation, a maximum of hundred and forty four images (i.e., 72 times 2) can be obtained from a single image acquired by the image detector.

Magnetic field generators 118 are firmly coupled with image detector 132. MPS sensor 112 is located at a distal portion (not shown) of a medical device 134. MPS sensor 114 is attached to a substantially stationary location of the body of patient 122. Medical device 134 is inserted to the body region of interest 120. MPS sensors 112 and 114, and magnetic field generators 118 are coupled with MPS 104. Each of MPS sensors 112 and 114 can be coupled with MPS 104 either by a conductor or by a wireless link. Processor 108 is coupled with moving imager 102, MPS 104, database 106 and with display 110.

Moving imager 102 is associated with an $X_{IMAGER}$, $Y_{IMAGER}$, $Z_{IMAGER}$ coordinate system (i.e., a 3D optical coordinate system). MPS 104 is associated with an $X_{MPS}$, $Y_{MPS}$, $Z_{MPS}$ coordinate system (i.e., a magnetic coordinate system). The scaling of the 3D optical coordinate system is different than that of the magnetic coordinate system. Moving mechanism 128 is coupled with moving assembly 126, thereby enabling moving assembly 126 to rotate about the $Y_{IMAGER}$ axis. Moving mechanism 128 rotates moving assembly 126 in directions designated by arrows 136 and 138, thereby changing the orientation of the radiation axis on the $X_{IMAGER}$-$Z_{IMAGER}$ plane and about the $Y_{IMAGER}$ axis. Moving mechanism 128 enables moving assembly 126 to rotate about the $X_{IMAGER}$ axis. Moving mechanism 128 rotates moving assembly 126 in directions designated by arrows 152 and 154, thereby changing the orientation of the radiation axis on the $Z_{IMAGER}$-$Y_{IMAGER}$ plane and about the $X_{IMAGER}$ axis. Moving imager 102 can include another moving mechanism (not shown) coupled with moving imager 102, which can move moving imager 102 along the $Y_{IMAGER}$ axis in directions designated by arrows 144 and 146 (i.e., along the cranio-caudal axis of patient 122). Moving imager 102 can include a further moving mechanism (not shown) coupled with moving imager 102, which can move moving imager 102 along the $X_{IMAGER}$ axis in directions designated by arrows 148 and 150 (i.e., perpendicular to the cranio-caudal axis of patient 122).

Moving mechanism 128 or another moving mechanism (not shown) coupled with operation table 124, can enable relative movements between moving imager 102 and the body region of interest 120 along the three axes of the 3D optical coordinate system, in addition to rotations in directions 136,138,152 and 154. Each of emitter 130 and image detector 132 is constructed and operative by methods known in the art.

Image detector 132 can be provided with linear motion in directions toward and away from emitter 130, for varying the focal length of the image (i.e., in order to zoom-in and zoom-out). This zoom operation is herein below referred to as "physical zoom". In this case, system 100 further includes a detector moving mechanism (not shown) coupled with image detector 132, in order to impart this linear motion to image detector 132. The detector moving mechanism can be either motorized or manual. The term "physical zoom" herein below, applies to an image detector which introduces distortions in an image acquired thereby (e.g., an image intensifier), as well as an image detector which introduces substantially no distortions (e.g., a flat detector). MPS sensor 116 (i.e., image detector MPS sensor) can be firmly coupled with image detector 132 and coupled with MPS 104, in order to detect the position of image detector 132 along an axis (not shown) substantially normal to the surface of emitter 130, in the magnetic coordinate system.

Alternatively, image detector 132 can include a position detector (not shown) coupled with processor 108, to inform processor 108 of the current position of moving imager 102 relative to emitter 130 . This position detector can be of a type known in the art, such as optic, sonic, electromagnetic, electric, mechanical, and the like. In case such a position detector is employed, processor 108 can determine the current position of moving imager 102 according to the output of the position detector, and MPS sensor 116 can be eliminated from system 100.

Alternatively, image detector 132 is substantially stationary relative to emitter 130 during the real-time operation of system 100. In this case, the physical zoom is performed by moving moving-assembly 126 relative to body region of interest 120, or by moving operation table 124. In this case, MPS sensor 116 can be eliminated from system 100. This arrangement is generally employed in mobile imagers, as known in the art. Alternatively, processor 108 can determine the physical zoom according to an input from the physical staff via the user interface. In this case too, MPS sensor 116 can be eliminated.

Additionally, moving imager 102 can perform a zoom operation which depends on an image detector ROI setting. In this case, an image processor (not shown) associated with moving imager 102, produces zoomed images of the acquired images, by employing different image detector ROI settings, while preserving the original number of pixels and the original dimensions of each of the acquired images.

It is noted that the physical zoom settings of image detector 132 is a substantially continuous function (i.e., the physical zoom can be set at any non-discrete value within a given range). The image detector ROI can be set either at one of a plurality of discrete values (i.e., discontinuous), or non-discrete values (i.e., continuous).

Magnetic field generators 118 are firmly coupled with image detector 132, in such a manner that magnetic field generators 118 do not physically interfere with radiations generated by image detector 132, and thus emitter 130 can direct a radiation at a field of view 140 toward the body region of interest 120, to be detected by image detector 132. In this manner, emitter 130 radiates a visual region of interest (not shown) of the body of patient 122. Image detector 132 produces an image output respective of the image of the body region of interest 120 in the 3D optical coordinate system. Image detector 132 sends the image output to processor 108 for display 110 to display the body region of interest 120.

Magnetic field generators 118 produce a magnetic field 142 toward the body region of interest 120, thereby magnetically radiating a magnetic region of interest (not shown) of the body of patient 122. Since magnetic field generators 118 are firmly coupled with image detector 132, the field of view 140 is included within magnetic field 142, no matter what the position of image detector 132. Alternatively, magnetic field 142 is included within field of view 140. In any case, body region of interest 120 is an intersection of field of view 140 and magnetic field 142. MPS 104 determines the position of the distal portion of medical device 134 (i.e., performs position measurements) according to the output of MPS sensor 112.

As a result of the direct and firm coupling of magnetic field generators 118 with image detector 132, the visual region of interest substantially coincides with the magnetic region of interest, and MPS sensor 112 responds to magnetic field 142 substantially at all times during the movements of moving imager 102. It is desirable to determine the position of the distal portion of medical device 134, while medical device 134 is inserted into any portion of the body of patient 122 and while moving imager 102 is imaging that same portion of the body of patient 122. Since magnetic field generators 118 are firmly coupled with moving imager 102 and move with it at all times, system 100 provides this capability. This is true for any portion of the body of patient 122 which moving imager 102 can move toward, in order to detect an image thereof.

Since magnetic field generators 118 are firmly coupled with moving imager 102, the 3D optical coordinate system and the magnetic coordinate system are firmly associated therewith and aligned together. Thus, when moving imager 102 moves relative to the body region of interest 120, magnetic field generators 118 move together with moving imager 102. The 3D optical coordinate system and the magnetic coordinate system are rigidly coupled. Therefore, it is not necessary for processor 108 to perform on-line computations for correlating the position measurements acquired by MPS 104 in the magnetic coordinate system, with the 3D optical coordinate system.

Thus, the position of MPS sensor 112 relative to the image of the body region of interest 120 detected by moving imager 102, can be determined without performing any real-time computations, such as transforming the coordinates according to a transformation model (i.e., transformation matrix), and the like. In this case, the transformation matrix for transforming a certain point in the magnetic coordinate system to a corresponding point in the 3D optical coordinate system, is a unity matrix.

It is noted that magnetic field generators 118 are located substantially close to that portion of the body of patient 122, which is currently being treated and imaged by moving imager 102. Thus, it is possible to use magnetic field generators which are substantially small in size and which consume substantially low electric power. This is true for any portion of the body of patient 122 which moving imager 102 can move toward, in order to detect an image thereof. This arrangement increases the sensitivity of MPS 104 to the movements of MPS sensor 112 within the body of patient 122, and reduces the cost, volume and weight of magnetic field generators 118.

Furthermore, this arrangement of magnetic field generators 118 provides the physical staff (not shown) a substantially clear view of body region of interest 120, and allows the physical staff a substantially easy reach to body region of interest 120. Since magnetic field generators 118 are firmly coupled with moving imager 102, any interference (e.g., magnetic, electric, electromagnetic) between MPS 104 and moving imager 102 can be identified beforehand, and compensated for during the operation of system 100.

It is further noted that the system can include MPS sensors, in addition to MPS sensor 112. It is noted that the magnetic field generators can be part of a transmitter assembly, which includes the magnetic field generators, a plurality of mountings for each magnetic field generator, and a housing to enclose the transmitter assembly components. The transmitter assembly can be for example, in an annular shape which encompasses image detector 132.

MPS 104 determines the viewing position value of image detector 132, according to an output of MPS sensor 114 (i.e., patient body MPS sensor), in the magnetic coordinate system, relative to the position of the body of patient 122. In this manner, processor 108 can compensate for the movements of patient 122 and of moving imager 102 during the medical operation on patient 122, according to an output of MPS 104, while processor 108 processes the images which image detector 132 acquires from body region of interest 120.

In case moving imager 102 is motorized, and can provide the position thereof to processor 108, directly, it is not necessary for processor 108 to receive data from MPS 104 respective of the position of MPS sensor 114, for determining the position of image detector 132. However, MPS sensor 114 is still necessary to enable MPS 104 to determine the position of the body of patient 122.

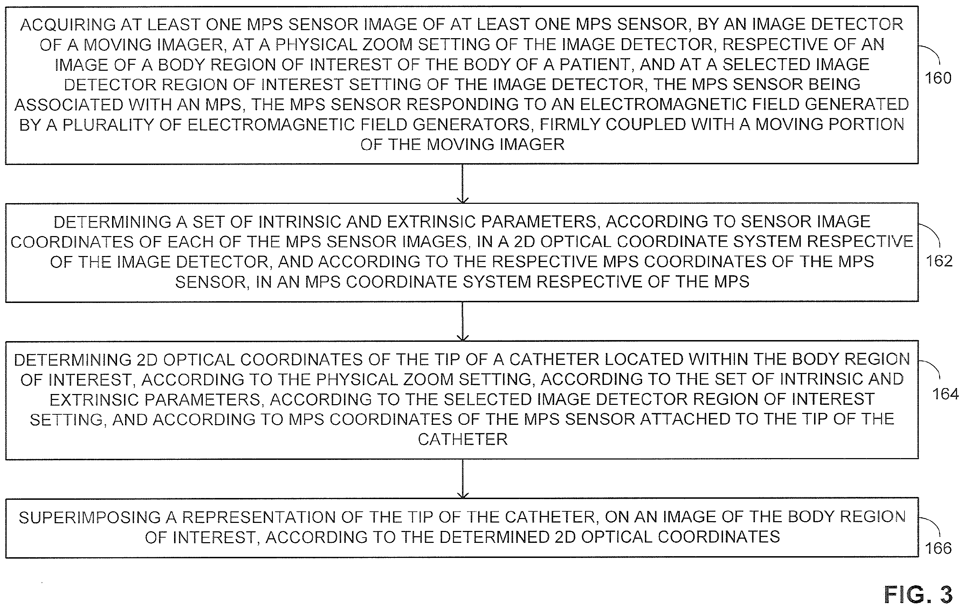

Reference is now made to Figure 3, which is a schematic illustration of a method for superimposing a representation of the tip of a medical device located within a body region of interest of the patient of Figure 2, on an image of the body region of interest, acquired by the image detector of the system of Figure 2, operative according to another embodiment of the disclosed technique. In procedure 160, at least one MPS sensor image of at least one MPS sensor, is acquired by an image detector of a moving imager, at a physical zoom setting of the image detector, respective of an image of a body region of interest of the body of a patient, and at a selected image detector region of interest setting of the image detector, the MPS sensor being associated with an MPS, the MPS sensor responding to an electromagnetic field generated by a plurality of electromagnetic field generators, firmly coupled with a moving portion of the moving imager.

With reference to Figure 2, an MPS sensor (not shown) is located within the field of view of image detector 132, and the MPS sensor is moved to different positions in space, while image detector 132 acquires a set of images of the MPS sensor. The MPS sensor can be mounted on a two-axis apparatus (not shown) for moving the MPS sensor in space. Alternatively, image detector 132 can acquire a single image of a plurality of MPS sensors.

This MPS sensor can be identical with MPS sensor 112. Alternatively, this MPS sensor can be identical with MPS sensor 114. Further alternatively, this MPS sensor can be different than either of MPS sensors 112 and 114.

Image detector 132 acquires the MPS sensor images, at one or more physical zoom settings of image detector 132, and at a selected image detector ROI setting of image detector 132. In case a plurality of different image detector ROI's are attributed to image detector 132, image detector 132 acquires the MPS sensor images at an image detector ROI setting, having the largest value. In case a single image detector ROI is attributed to image detector 132, the MPS sensor images which image detector acquires from the MPS sensor, is attributed to this single image detector ROI.

Magnetic field generators 118 (i.e., MPS transmitters) are firmly coupled with image detector 132, at a periphery thereof. Image detector 132 is associated with the 3D optical coordinate system, whereas magnetic field generators 118 are associated with the magnetic coordinate system of MPS 104. It is noted that the magnetic coordinate system and the 3D optical coordinate system, are arbitrarily set to be substantially identical, such that they share the same origin and the same axes in space. The magnetic coordinate system is employed as the frame of reference for either of MPS sensors 112, 114, and 116, and the 3D optical coordinate system can be referred to this magnetic coordinate system. The MPS sensor responds to the electromagnetic field generated by electromagnetic field generators 118, by producing an output according to the position of the MPS sensor relative to electromagnetic field generators 118, in the magnetic coordinate system.

In procedure 162, a set of intrinsic and extrinsic parameters is determined, according to sensor image coordinates of each of the MPS sensor images, in a 2D optical coordinate system respective of the image detector, and according to the respective MPS coordinates of the MPS sensor, in an MPS coordinate system respective of the MPS. The intrinsic parameters of image detector 132 depend on the physical zoom setting of image detector 132, no matter whether image detector 132 introduces distortions in the image acquired thereby, or not (e.g., both in case of an image intensifier and a flat detector, respectively). The intrinsic parameters are represented by a matrix **M**.

Processor 108 determines the intrinsic parameters at each of the physical zoom settings of image detector 132. Processor 108 can determine the intrinsic and extrinsic parameters at a selected physical zoom setting, either by interpolating between two adjacent physical zoom settings, or by extrapolating there between. For example, if intrinsic and extrinsic parameters for image detector 132 at physical zoom settings of 15.1, 15.3, and 15.7, are stored in processor 108, and an intrinsic and extrinsic parameter is to be determined at physical zoom setting of 15.2, then processor 108 determines these intrinsic and extrinsic parameters, by interpolating between physical zoom settings of 15.1 and 15.3. On the other hand, if intrinsic and extrinsic parameters are to be determined at physical zoom setting of 15.9, then processor 108 determines these intrinsic and extrinsic parameters, by extrapolating between physical zoom settings of 15.3 and 15.7. If intrinsic and extrinsic parameters are available at only two physical zoom settings (e.g., two extreme positions of image detector 132), then processor 108 can either interpolate or extrapolate between these two physical zoom settings.

Processor 108 can determine the intrinsic parameters more accurately, the more images image detector 132 acquires from the MPS sensor, at different physical zoom settings of image detector 132. Alternatively, processor 108 can determine the intrinsic parameters according to only two images acquired by image detector 132, at two extreme physical zoom settings of image detector 132.

In case image detector 132 introduces substantially no distortions in the image which image detector 132 acquires (e.g., in case of a flat detector), the intrinsic parameters are influenced in a substantially linear manner, by the physical zoom setting of image detector 132. However, in case image detector 132 introduces distortions in the image due to viewing position distortions (e.g., in case of an image intensifier), the intrinsic parameters are influenced by the physical zoom setting, in a random manner. Therefore, in case of an image intensifier, processor 108 determines the intrinsic parameters according to the physical zoom settings and the viewing position of image detector 132.

The extrinsic parameters define the rotation and translation of image detector 132 relative to the magnetic coordinate system (i.e., the extrinsic parameters represent the mechanical connection between electromagnetic field generators 118, and moving imager 102). The extrinsic parameters remain the same, regardless of any change in the physical zoom setting of image detector 132, or in the setting of the image detector region of interest of image detector 132, unless the mechanical coupling between electromagnetic field generators 118 and image detector 132 is modified. The extrinsic parameters can be represented either as a constant matrix **N**, or as a constant multiplier embedded in the intrinsic parameters.

Processor 108 determines the intrinsic and extrinsic parameters, according to the coordinates of each of the MPS sensor images which image detector 132 acquires in procedure 160, in the 2D optical coordinate system of image detector 132, and according to the respective coordinates of the same MPS sensor, in the magnetic coordinate system of MPS 104. In case image detector 132 acquires a single MPS sensor image of a plurality of MPS sensors, processor 108 determines the intrinsic and extrinsic parameters, according to the coordinates of each of the MPS sensors, in the 2D optical coordinate system of image detector 132, and according to the coordinates of the respective MPS sensors in the magnetic coordinate system of MPS 104.

In procedure 164, 2D optical coordinates of the tip of a catheter located within the body region of interest is determined, according to the physical zoom setting, according to the set of intrinsic and extrinsic parameters, according to the image detector region of interest setting, and according to MPS coordinates of the MPS sensor attached to the tip of the catheter. With reference to Figure 2, the 2D optical coordinates of the tip of catheter 134 is represented by a vector **L**. The real-time magnetic coordinates of MPS sensor 112 is represented by a vector **Q**. The connection between the magnetic coordinate system of MPS 104, and the 3D optical coordinate system of image detector 132 is represented by a matrix **R**.

In case of system 100, where magnetic field generators 118 are coupled with image detector 132, the magnetic coordinate system and the 3D optical coordinate system are associated with a common origin and orientation (without loss of generality), and therefore it is not necessary to determine the connection there between. Therefore, **R**=1. The intrinsic parameters are represented by a matrix **M**, and the extrinsic parameters by a matrix **N**. The 2D optical coordinates of the tip of catheter 134 are determined according to,

$$L = MNRQ \qquad (1)$$

with $R \neq 1$. In case of Figure 2, where $R = 1$, the 2D optical coordinates of the tip of catheter 134 are determined according to,

$$L = MNQ \qquad (2)$$

and in case the extrinsic parameters are included in the intrinsic parameters, the 2D optical coordinates of the tip of catheter 134 are determined according to,

$$L = MQ \qquad\qquad (3)$$

Processor 108 determines the 2D optical coordinates of the tip of catheter 134, according to the physical zoom settings of image detector 132, according to the set of the intrinsic and extrinsic parameters of image detector 132, as determined in procedure 162, according to the image detector region of interest setting, and according to the coordinates of MPS sensor 112 in the MPS coordinate system of MPS 104.

In procedure 166, a representation of the tip of the catheter is superimposed on an image of the body region of interest, according to the determined 2D optical coordinates. With reference to Figure 2, Processor 108 superimposes a representation of the 2D optical coordinates determined in procedure 164, on an image of body region of interest 120. It is noted that the image of body region of interest 120 is distorted due to the intrinsic parameters and the extrinsic parameters of image detector 132, and possibly due to image rotation, image flip, viewing position of image detector 132, and scaling of the image, depending on the type of image detector employed in system 100 (i.e., whether image detector 132 introduces distortions to the image or not). Display 110 displays this superposition on the image of body region of interest 120, and the physical staff can obtain substantially accurate information respective of the position of the tip of catheter 134, within body region of interest 120.

It is noted that the method according to Figure 3, concerns an image detector which includes a single image detector ROI. In case image detector 132 is provided with a plurality of image detector regions of interest, a scale function between different image detector regions of interests is determined, by employing a full span fiducial screen, and by performing the following procedures before performing procedure 160.

Initially, the full span fiducial screen is located in a field of view of image detector 132, such that the image acquired by image detector 132, includes the image of the fiducials of the full span fiducial screen. This full span fiducial screen can be constructed for example, from a transparent material (e.g., plastic sheet) in which translucent markers (e.g., steel balls), are embedded therein. Such a full span fiducial screen can include tens of markers which are dispersed on a rectilinear grid on the entire surface of the full span screen.

Next, a plurality of marker images is acquired by image detector 132, at different image detector regions of interest, at a selected physical zoom setting (i.e., a constant physical zoom setting), wherein each of the marker images includes an image of the fiducials. Next, processor 108 determines a scale function **s** between the different image detector regions of interest, according to the coordinates of the fiducials in each of the marker images (i.e., the marker image coordinates), and according to the actual coordinates of the respective fiducials. In this case, processor 108 determines the 2D optical coordinates of the tip of catheter 134, according to,

$$L = sMNRQ \qquad\qquad (4)$$

In case image detector 132 scales an image up and down in a uniform manner, about the center of the image while producing substantially no distortions (e.g., in case of a flat detector), then the scale function **s** is treated as a scale factor (i.e., a rational number). However, in case image detector 132 scales the image up and down in a non-uniform manner (e.g., in case of an image intensifier), each scaled image is further distorted in a different manner, and then a scale function is employed. In this case, the scale function is also affected by the physical zoom setting and the viewing position of image detector 132 as described herein below.

Once processor 108 determines the scale function, the full span fiducial screen can be removed from the field of view of image detector 132, and the method can be resumed starting at procedure 160.

It is noted that procedure 162 applies to an image detector which introduces substantially no viewing position distortions in the image acquired by image detector 132 (e.g., in case of a flat detector). In case image detector 132 introduces viewing position distortions (e.g., in case of an image intensifier), the method includes a further procedure of determining a viewing position transformation model, in order to take into account the viewing position distortion, when performing procedure 162.

For this purpose, a peripheral fiducial screen is firmly coupled with image detector 132, in front of image detector 132, in an off-line mode of operation of system 100 (i.e., before performing the medical operation on patient 122). This peripheral fiducial screen is of such a form that the images (i.e., peripheral marker images) of the fiducials (i.e., peripheral fiducials) fall on a periphery of an image of body region of interest 120. Each fiducial in a group of fiducials is complementary to the rest of the fiducials in that group, such that if processor 108 is unable to identify one or more fiducials in the image acquired by image detector 132 (e.g., the fiducial is located in a dark portion of the image), then processor 108 can still determine the coordinates of the rest of the fiducials according to the coordinates of at least one fiducial which is clearly recognizable. This is provided by arranging the fiducials in a predetermined geometry, for example by employing fiducials of predetermined unique shapes and sizes, predetermined patterns of fiducials, and the like. The geometry of the peripheral fudicial screen conforms to the geometry of the image detected by image detector 132, such as circular, chamfered corners, round corners, and the like.

After mounting the peripheral fiducial screen in front of image detector 132, image detector 132 acquires a reference image at a reference position (e.g., 0, 0, 0 in the 3D optical coordinate system), in a non-real-time mode of operation, at each image detector ROI setting, and at each of the physical zoom settings of image detector 132. For example, if image detector 132 includes three image detector ROI settings, and three physical zoom settings, then image detector 132 acquires a total of nine reference images. The reference image includes the peripheral marker images. Processor 108 determines the scale function **s** for each combination of different image detector ROI settings, and different physical zoom settings, in the non-real-time mode of operation. Processor 108 determines the viewing position transformation model in real-time, according to the coordinates of the peripheral fiducials in the reference image, which image detector 132 acquires off-line, and according to the coordinates of the peripheral fiducials in an image which image detector 132 acquires in real-time with respect to the physical zoom setting and the image detector ROI setting thereof. Processor 108 performs procedure 164, furthermore, according to this viewing position transformation model.

Alternatively, processor 108 determines a plurality of viewing position transformation models, corresponding to respective viewing position values of image detector 132, in the non-real-time mode of operation of system 100, according to fiducial image coordinates of the peripheral fiducials in the peripheral marker images, and according to the actual coordinates of the peripheral fiducials of the peripheral fiducial screen. Processor 108 constructs a logical relationship between each viewing position transformation model, and the respective viewing position value, in the non-real-time mode of operation of system 100. In the real-time mode of operation of system 100, processor 108 receives information respective of the viewing position value of image detector 132.

Processor 108 can receive this information either from image detector 132 itself, or from a user interface (not shown), coupled with image detector 132. Processor 108 determines the viewing position transformation model, corresponding to the respective viewing position value, contained in the received information, according to the logical relationship, in real-time. Processor 108 performs procedure 164, furthermore, according to this viewing position transformation model.

It is noted that procedure 162 applies to an image detector which introduces substantially no image flip distortion or image rotation distortion to an image acquired thereby (e.g., in case of a flat detector), when the image is rotated or flipped. In case image detector 132 introduces image flip distortion and image rotation distortion (e.g., in case of an image intensifier), the method includes a further procedure of determining an image rotation correction model and an image flip correction model. Processor 108, then determines the 2D optical coordinates of the tip of catheter 134, according to the image rotation correction model and the image flip correction model, as well as the intrinsic parameters, the extrinsic parameters, the physical zoom settings, the image detector ROI settings, and the real-time coordinates of MPS sensor 112.

The image rotation correction model is a model (e.g., a transformation matrix), which processor 108 utilizes to determine the 2D optical coordinates of the tip of catheter 134, in procedure 164. The image rotation correction model can involve a rotation distortion which image detector 132 introduces in the image which image detector 132 acquires (e.g., in case image detector 132 is an image intensifier, and where the rotation is performed on an analog image acquired by image detector 132). In this case, while processor 108 utilizes the image rotation correction model in performing procedure 164, processor 108 takes into account the distortion in the image acquired by image detector 132, due to the rotation of the image, as well as the changes in the coordinates of the image in the 2D optical coordinate system, due to the sheer action of rotation. The same argument applies to an image flip process.

It is noted that in case the image rotation is performed on a digital image (i.e., by digitizing the analog image which image detector 132 acquires), the image rotation correction model excludes any image rotation distortion, and procedure 164 involves only transformation due to the rotation procedure per se, and excludes any correction due to image rotation distortion. The same argument holds with regard to an image flip process.

Processor 108 determines the real-time image rotation distortion and the real-time image flip distortion according to a logical relationship (e.g., a look-up table, a mathematical function), which processor 108 constructs off-line, and stores this logical relationship in database 106. For this purpose the peripheral fiducial screen described herein above, is firmly coupled with image detector 132, in front of image detector 132.

Processor 108 associates the amount of each image rotation and image flip, of a reference image which image detector 132 acquires at the reference position at different physical zoom settings and different image detector ROI settings, with the respective pattern of the peripheral fiducials in the reference image, and enters this association in the look-up table. Processor 108 determines each image rotation correction model and each image flip correction model, of the respective image rotation and image flip, according to the pattern of the peripheral fiducials in the reference image and the actual pattern of the peripheral fiducials in the peripheral fiducial screen, and enters the data respective of these distortions in the look-up table. Processor 108, furthermore determines the real-time image rotation distortion and the real-time image flip distortion, associated with a real-time image of body region of interest 120, by referring to the look-up table, and by determining the unique pattern of the peripheral fiducials of the peripheral fiducial screen, in the real-time image which image detector 132 acquires.

It is noted that processor 108 employs the look-up table to determine the 2D optical coordinates of the tip of catheter 134, according to the coordinates of the peripheral fiducials, while leaving the distorted real-time image intact, thereby saving precious processing time and central processing unit (CPU) resources.

It is further noted that in case moving imager 102 is capable to notify processor 108 of the current image rotation value and the image flip value, processor 108 can determine the image rotation correction model and the image flip correction model, according to this information, and use this information according to the look-up table in real-time. This is true both in case image detector 132 introduces distortions in the image acquired thereby (e.g., in case of an image intensifier), and in case image detector 132 introduces substantially no distortions (e.g., in case of a flat detector). Alternatively, processor 108 can determine the current image rotation value and the current image flip value, according to the relevant data that the physical staff enters via the user interface.

In case image detector 132 introduces substantially no distortions in the image acquired thereby (e.g., in case of a flat detector), due to an image rotation operation, processor 108 can determine the image rotation correction model according to the value of the image rotation, and according to the look-up table. Processor 108 determines the image rotation correction model in real-time, according to the coordinates of the peripheral fiducials in the reference image, which image detector 132 acquires off-line, and according to the coordinates of the peripheral fiducials in an image which image detector 132 acquires in real-time with respect to the physical zoom setting and the image detector ROI setting thereof. Processor 108 takes into account this image rotation correction model, while performing procedure 164, as described herein above. In this case, the image rotation correction model pertains to a change in the 2D optical coordinates of the image due to the rotation operation alone, and precludes any image rotation distortion due to the image rotation operation. The same argument holds with respect to an image flip operation.

In case image detector 132 introduces distortions in an image acquired thereby (e.g., in case of an image intensifier), as a result of a change in scale, processor 108 takes into account this scale function for determining the 2D optical coordinates of the tip of catheter 134, as described herein above in connection with procedure 164. One of the following scenarios can prevail, while the physical staff operates system 100:

- Superimposing a real-time representation of the tip of catheter 134 on a real-time image of body region of interest 120. In this case, if MPS sensor 112 produces an output at a time $t_{PNO}$, and the image of body region of interest 120 is associated with a time $t_{IMACE}$, then $t_{PNO} = t_{IMAGE}$. Since the magnetic coordinate system and the 3D optical coordinate system are by definition substantially identical, processor 108 can determine the relation between the coordinates of MPS sensor 112, and the coordinates of every pixel in the image acquired by image detector 132, according to Equation (1). Since MPS sensor 112 moves together with the body of patient 122, MPS sensor 112 detects the movements of the body of patient 122, and MPS sensor 114 can be eliminated from system 100.

- Superimposing a real-time representation of the tip of catheter 134 on a non-real-time image of body region of interest 120 (i.e., an image which image detector 132 has acquired from body region of interest 120, during the medical operation on patient 122, and a substantially short while ago, for example, several minutes before determination of the position of the tip of catheter 134, by processor 108). This non-real time image of body region of interest 120, can be either a still image, or a cine-loop (i.e., a video clip). In this case $t_{PNO} > t_{IMAGE}$, and MPS sensor 114 is required for system 100 to operate. Processor 108 determines the 2D optical coordinates of the tip of catheter 134, according to the coordinates of MPS sensor 112 at time $t_{PNO}$ (i.e., in real-time) and the coordinates of MPS sensor 114 at time $t_{IMAGE}$ which is associated with the non-real time image acquired by image detector 132 at time $t_{IMAGE}$ (i.e., an image acquired during the medical operation on patient 122, a short while ago).

- Superimposing a non-real-time representation of the tip of catheter 134 on a real-time image of body region of interest 120 acquired by image detector 132 (i.e., $t_{PNO} < t_{IMAGE}$). In this case processor 108 determines the 2D coordinates of the tip of catheter 134 according to the coordinates of MPS sensor 112 at time $t_{PNO}$ (i.e., processor 108 has determined the 2D coordinates of the tip of catheter 134 during the medical operation on patient 122, and a short while ago, for example, several minutes before acquisition of the image of body region of interest 120 by image detector 132), and according to the coordinates of MPS sensor 114 at time $t_{IMAGE}$ (i.e., in real-time). In this case too, MPS sensor 114 is required for system 100 to operate.

- Superimposing a non-real-time representation of the tip of catheter 134 on a non-real-time image of body region of interest 120 acquired by image detector 132 (i.e., $t_{PNO} \neq t_{IMAGE}$). In this case processor 108 determines the 2D coordinates of the tip of catheter 134 according to the coordinates of MPS sensor 112 at time $t_{PNO}$ (i.e., still during the same medical operation on patient 122), and according to the coordinates of MPS sensor 114 at time $t_{IMAGE}$ (i.e., still during the same medical operation on patient 122). In this case too, MPS sensor 114 is required for system 100 to operate.

It is noted that the combinations of real-time, and non-real-time representation of the tip of catheter 134, and real-time and non-real-time image of body region of interest 120, enables the physical staff to investigate previous instances of the tip of catheter 134 and body region of interest 120, during the same operation on patient 122. For example, by providing a display of a superimposition of a real-time representation of the tip of catheter 134 on a non-real-time image of body region of interest 120, the physical staff can observe a superimposition of the current position of the tip of catheter 134, on body region of interest 120, without having to expose patient 122, the physical staff, or both, to harmful radioactive waves.

Reference is now made to Figure 4, which is a schematic illustration of a system, generally referenced 200, for determining the position of the tip of a medical device relative to images of the body of a patient detected by a moving imager, the system being constructed and operative in accordance with a further embodiment of the disclosed technique. System 200 includes a moving imager 202, an MPS sensor 204, an MPS 206 and a plurality of magnetic field generators 208.

Moving imager 202 includes a moving assembly 210, a moving mechanism 212, an image detector 214 and an emitter 216. The movements of moving imager 202 are similar to those of moving imager 102 (Figure 2) as described herein above.

Image detector 214 and emitter 216 are coupled with moving assembly 210, such that image detector 214 is located on one side of a patient 218, and emitter 216 is located at the opposite side of patient 218. Image detector 214 and emitter 216 are located on a radiation axis (not shown), wherein the radiation axis crosses a body region of interest 220 of patient 218. Patient 218 is lying on an operation table 222.

A medical device 224 is inserted into the body region of interest 220. MPS sensor 204 and magnetic field generators 208 are coupled with MPS 206. MPS sensor 204 is located at a distal portion of medical device 224.

Image detector 214 directs a radiation at a field of view 226 toward the body region of interest 220, to be detected by emitter 216, thereby radiating a visual region of interest (not shown) of the body of patient 218. Magnetic field generators 208 produce a magnetic field 228 in a magnetic region of interest (not shown) of the body of patient 218. Since magnetic field generators 208 are firmly coupled with moving imager 202, the magnetic region of interest substantially coincides with the visual field of interest substantially at all positions and orientations of moving imager 202 relative to the body of patient 218. Hence, MPS 206 can determine the position of MPS sensor 204 relative to an image of the body of patient 218 which moving imager 202 images. This is true for substantially all portions of the body of patient 218 which moving imager 202 is capable to image. Magnetic field generators 208 can be housed in a transmitter assembly (not shown) which is firmly coupled with emitter 216 (e.g., located beside the emitter).

It is noted that the magnetic field generators can be firmly coupled with a portion of the moving assembly between the image detector and the emitter. In this case too, the magnetic region of interest substantially coincides with the visual region of interest, and the MPS is capable to determine the position of the MPS sensor at substantially all positions and orientations of the moving imager. In any case, the magnetic field generators are firmly coupled with that moving portion of the moving imager, which moves together with those elements of the moving imager, which are involved in imaging the body region of interest (e.g., the image detector and the emitter).

Reference is now made to Figure 5, which is a schematic illustration of a system, generally referenced 250, for determining the position of the tip of a medical device relative to images of the body of a patient detected by a computer assisted tomography (CAT) machine, the system being constructed and operative in accordance with another embodiment of the disclosed technique. System 250 includes a CAT machine (not shown), an MPS sensor 252, an MPS 254, and a plurality of magnetic field generators 256. The CAT machine includes a revolving portion 258, and a slidable bed 260. Revolving portion 258 can revolve about a longitudinal axis 262 of the CAT machine and of slidable bed 260, in clockwise and counterclockwise directions 264 and 266, respectively, as viewed along longitudinal axis 262. Revolving portion 258 includes an emitter 262 and an image detector 264, located opposite one another along a plane (not shown) of revolving portion 258, substantially perpendicular to longitudinal axis 262.

Magnetic field generators 256 can be housed in a transmitter assembly (not shown) which is firmly coupled with emitter 262 (e.g., located beside the emitter, or in a periphery thereof). A medical device 268 is inserted into the body region of interest 270 of a patient 272 who is lying on slidable bed 260. MPS sensor 252 and magnetic field generators 256 are coupled with MPS 254. MPS sensor 252 is located at a distal portion of medical device 268. Emitter 262 emits X-rays toward image detector 264 through body region of interest 270, for image detector 264 to detect an image (not shown) of body region of interest 270.

MPS sensor 252 produces an output when magnetic field generators 256 emit a magnetic field toward body region of interest 270, and MPS 254 determines the position of the tip of medical device 268, according to the output of MPS sensor 252. Alternatively, the magnetic field generators can be coupled with the image detector.

It will be appreciated by persons skilled in the art that the disclosed technique is not limited to what has been particularly shown and described hereinabove. Rather the scope of the disclosed technique is defined only by the claims, which follow.

## Claims

1. Method for displaying a representation of the tip of a medical device located within a body region of interest (120) of the body of a patient (122), on an image of the body region of interest, the image being acquired by an image detector (132) of a moving imager (102) at a selected physical zoom setting, the method comprising:

   acquiring at least one medical positioning system (MPS) sensor image of an MPS sensor, by said image detector (132), at one or more physical zoom settings of said image detector (132) respective of said image, and at a selected image detector region of interest setting of said image detector (132), said MPS sensor being associated with an MPS (104), said at least one MPS sensor (112, 114, 116) responding to an electromagnetic field generated by a plurality of electromagnetic field generators (118), that are firmly coupled with a moving portion of said moving imager (102);
   determining a set of intrinsic and extrinsic parameters at the selected physical zoom setting, according to sensor image coordinates of each of the MPS sensor images, in a two-dimensional optical coordinate system respective of said image detector (132), and according to non-real-time MPS coordinates of said MPS sensor (112, 114, 116), in an MPS coordinate system respective of said MPS, wherein each set of intrinsic parameters comprises optical characteristics of said image detector and each set of extrinsic parameters comprises rotation and translation parameters of the MPS coordinate system relative to the optical coordinate system;
   determining two-dimensional coordinates of said tip of said medical device, according to said selected physical zoom setting, according to said set of intrinsic and extrinsic parameters, according to said selected image detector region of interest setting, and according to real-time MPS coordinates of an MPS sensor located at said tip of said medical device;
   superimposing said representation of said tip of said medical device, on said image of said body region of interest, according to said two-dimensional coordinates; and
   displaying said representation of said tip of said medical device superimposed on said image of said body region of interest, wherein the method is **characterised in that**

   said step of determining said set of intrinsic and extrinsic parameters is performed with respect to a plurality of physical zoom settings of said image detector, by interpolating between intrinsic and extrinsic parameters associated with two adjacent ones of said physical zoom settings or by extrapolating beyond intrinsic and extrinsic parameters associated with two adjacent ones of said physical zoom settings.

2. The method according to claim 1, further comprising a step of removing a full span fiducial screen from a field of view of said image detector, said full span fiducial screen having been placed in said field of view, in an off-line mode of operation of a system operating according to said method, said full span fiducial screen including a plurality of fiducials, comprising at least one group of complementary fiducials wherein every fiducial in a group is complementary to the rest of said fiducials in said group.

3. The method according to claim 2, wherein said selected image detector region of interest setting is one of one or more region of interest settings corresponding to one or more regions of interest, further comprising a step of determining a scale function between different image detector regions of interest, according to fiducial image coordinates of said at least one group of complementary fiducials in a plurality of fiducial images acquired by said image detector at said different image detector regions of interest, and at said selected physical zoom setting, and according to actual coordinates of said complementary fiducials.

4. The method according to claim 3, wherein said procedure of determining said two-dimensional coordinates is performed according to said scale function.

5. The method according to claim 3, further comprising a step of acquiring said fiducial images by said image detector (132).

6. The method according to claim 5, further comprising a step of placing said full span fiducial screen in said field of view.

7. The method according to claim 1, wherein said at least one MPS sensor image includes a single MPS sensor image of a plurality of MPS sensors.

8. The method according to claim 1, wherein said selected image detector region of interest is the largest image detector region of interest, among a plurality of image detector regions of interest.

9. The method according to claim 1, further comprising a step of:

firmly attaching a peripheral fiducial screen to said image detector, in front of said image detector, in a non-real-time mode of operation of a system operating according to said method, said peripheral fiducial screen including a plurality of peripheral fiducials, comprising at least one group of complementary fiducials wherein every fiducial in a group is complementary to the rest of said peripheral fiducials in said group;

acquiring at least one reference image of said body region of interest, by said image detector in said non-real-time mode of operation of said system, at a reference position of said moving imager, at each physical zoom setting of said image detector, and at each image detector region of interest setting of said image detector, each of said at least one reference image including a plurality of peripheral fiducial images of said peripheral fiducials, at a periphery of said at least one reference image; and

determining a viewing position transformation model respective of a viewing position of said image detector, in a real-time mode of operation of said system, according to a first set of coordinates of said peripheral fiducials in said at least one reference image, and according to a second set of coordinates of said peripheral fiducials, in a real-time image of said body region of interest acquired by said image detector.

10. The method according to claim 9, wherein said step of determining said coordinates of said tip of said medical device, is performed furthermore according to said viewing position transformation model.

11. The method according to claim 9, further comprising:

determining a set of image rotation correction models respective of said first set of coordinates, in each of said at least one reference image, in a non-real-time mode of operation of said system;

constructing a logical relationship between each image rotation correction model of said set of image rotation correction models, and said respective first set of coordinates, in said non-real-time mode of operation of said system;

determining an image rotation correction model corresponding to said second set of coordinates, according to said logical relationship, in a real-time mode of operation of said system; and

performing said procedure of determining said two-dimensional optical coordinates of said tip of said medical device, furthermore according to said image rotation correction model.

12. The method according to claim 9, further comprising:

determining a set of image flip correction models respective of said first set of coordinates, in each of said at least one reference image, in a non-real-time mode of operation of said system;

constructing a logical relationship between each image flip correction model of said set of image rotation correction models, and said respective first set of coordinates, in said non-real-time mode of operation of said system;

determining an image flip correction model corresponding to said second set of coordinates, according to said logical relationship, in a real-time mode of operation of said system; and

performing said procedure of determining said two-dimensional coordinates of said tip of said medical device, furthermore according to said image flip correction model.

13. The method according to claim 1, further comprising:

determining a plurality of viewing position distortion models corresponding to respective ones of a plurality of viewing position values of said image detector, in a non-real-time mode of operation of a system operating according to said method,

constructing a first logical relationship between said viewing position distortion models, and said respective viewing position values, in said non-real-time mode of operation of said system;

receiving information respective of a viewing position value of said image detector, from a user interface, in a real-time mode of operation of said system; and

determining a viewing position distortion model corresponding to said viewing position value, according to said first logical relationship, in said real-time mode of operation of said system.

**14.** The method according to claim 13, wherein said step of determining said two-dimensional coordinates of said tip of said medical device, is performed furthermore according to said viewing position transformation model.

**15.** The method according to claim 13, further comprising:

determining a plurality of image rotation correction models corresponding to respective ones of a plurality of image rotation values of another image of said body region of interest, in said non-real-time mode of operation of said system,
constructing a second logical relationship between said image rotation correction models and said respective image rotation values, in said non-real-time mode of operation of said system;
receiving information respective of an image rotation value of said image, from a user interface, in a real-time mode of operation of said system; and
determining an image rotation correction model corresponding to said image rotation value, according to said second logical relationship, in said real-time mode of operation of said system.

**16.** The method according to claim 13, further comprising:

determining a plurality of image flip correction models corresponding to respective ones of a plurality of image flip values of another image of said body region of interest, in said non-real-time mode of operation of said system,
constructing a second logical relationship between said image flip correction models and said respective image flip values, in said non-real-time mode of operation of said system;
receiving information respective of an image flip value of said image, from a user interface, in a real-time mode of operation of said system; and
determining an image flip correction model corresponding to said image flip value, according to said second logical relationship, in said real-time mode of operation of said system.

**17.** The method according to claim 1, further comprising:

firmly attaching a peripheral fiducial screen to said image detector, in front of said image detector, in a non-real-time mode of operation of a system operating according to said method, said peripheral fiducial screen including a plurality of peripheral fiducials comprising at least one group of complementary peripheral fiducials, wherein every peripheral fiducial in a group is complementary to the rest of said peripheral fiducials in said group;
acquiring at least one reference image of said body region of interest, by said image detector in said non-real-time mode of operation of said system, at a reference position of said moving imager, at each physical zoom setting of said image detector, and at each image detector region of interest setting of said image detector, each of said at least one reference image including a plurality of peripheral fiducial images of said peripheral fiducials, at a periphery of said at least one reference image;
determining a plurality of viewing position transformation models corresponding to respective ones of a plurality of viewing position values of said image detector, in a non-real-time mode of operation of a system operating according to said method, according to fiducial image coordinates of said peripheral fiducials in respective ones of said at least one reference image, and according to actual coordinates of said peripheral fiducials;
constructing a first logical relationship between said viewing position transformation models, and said respective viewing position values, in said non-real-time mode of operation of said system;
receiving information respective of a viewing position value of said image detector, from said image detector, in a real-time mode of operation of said system; and
determining a viewing position transformation model corresponding to said viewing position value, according to said first logical relationship, in said real-time mode of operation of said system.

**18.** The method according to claim 17, wherein said step of determining said two-dimensional optical coordinates of said tip of said medical device, is performed furthermore according to said viewing position transformation model.

**19.** The method according to claim 17, further comprising:

determining a plurality of image rotation correction models corresponding to respective ones of a plurality of image rotation values of another image of said body region of interest, in said non-real-time mode of operation of said system;

constructing a second logical relationship between said image rotation correction models and said respective image rotation values, in said non-real-time mode of operation of said system;

receiving information respective of an image rotation value of said image, from said image detector, in a real-time mode of operation of said system; and

determining an image rotation correction model corresponding to said image rotation value, according to said second logical relationship, in said real-time mode of operation of said system.

**20.** The method according to claim 17, further comprising:

determining a plurality of image flip correction models corresponding to respective ones of a plurality of image flip values of another image of said body region of interest, in said non-real-time mode of operation of said system;

constructing a second logical relationship between said image flip correction models and said respective image flip values, in said non-real-time mode of operation of said system;

receiving information respective of an image flip value of said image, from said image detector, in a real-time mode of operation of said system; and

determining an image flip correction model corresponding to said image flip value, according to said second logical relationship, in said real-time mode of operation of said system.

**21.** The method according to claim 1, further comprising:

firmly attaching a peripheral fiducial screen to said image detector, in front of said image detector, in a non-real-time mode of operation of a system operating according to said method, said peripheral fiducial screen including a plurality of peripheral fiducials comprising at least one group of complementary fiducials, wherein every peripheral fiducial in a group is complementary to the rest of said peripheral fiducials in said group;

acquiring at least one reference image of said body region of interest, by said image detector in said non-real-time mode of operation of said system, at a reference position of said moving imager, at each physical zoom setting of said image detector, and at each image detector region of interest of said image detector, each of said at least one reference image including a plurality of peripheral fiducial images of said peripheral fiducials, at a periphery of said at least one reference image; and

determining an image rotation correction model respective of an image rotation value of said image, in a real-time mode of operation of said system, according to a first set of coordinates of said peripheral fiducials in said at least one reference image, and according to a second set of coordinates of said peripheral fiducials, in said image.

**22.** The method according to claim 21, wherein said step of determining said two-dimensional coordinates of said tip of said medical device, is performed furthermore according to said image rotation correction model.

**23.** The method according to claim 1, further comprising:

firmly attaching a peripheral fiducial screen to said image detector, in front of said image detector, in a non-real-time mode of operation of a system operating according to said method, said peripheral fiducial screen including a plurality of peripheral fiducials comprising at least one group of complementary fiducials, wherein every peripheral fiducial in a group is complementary to the rest of said peripheral fiducials in said group;

acquiring at least one reference image of said body region of interest, by said image detector in said non-real-time mode of operation of said system, at a reference position of said moving imager, at each physical zoom setting of said image detector, and at each image detector region of interest of said image detector, each of said at least one reference image including a plurality of peripheral fiducial images of said peripheral fiducials, at a periphery of said at least one reference image; and

determining an image flip correction model respective of an image rotation value of said image, in a real-time mode of operation of said system, according to a first set of coordinates of said peripheral fiducials in said at least one reference image, and according to a second set of coordinates of said peripheral fiducials, in said image.

**24.** The method according to claim 23, wherein said step of determining said two-dimensional coordinates of said tip of said medical device, is performed furthermore according to said image flip correction model.

**25.** The method according to claim 1, further comprising:

determining a plurality of image rotation correction models corresponding to respective ones of a plurality of image rotation values of another image of said body region of interest, in said non-real-time mode of operation of said system;

constructing a logical relationship between said image rotation correction models and said respective image rotation values, in said non-real-time mode of operation of said system;

receiving information respective of an image rotation value of said image, from a user interface, in a real-time mode of operation of said system; and

determining an image rotation correction model corresponding to said image rotation value, according to said logical relationship, in said real-time mode of operation of said system.

26. The method according to claim 1, further comprising:

determining a plurality of image flip correction models corresponding to respective ones of a plurality of image flip values of another image of said body region of interest, in said non-real-time mode of operation of said system;

constructing a logical relationship between said image flip correction models and said respective image flip values, in said non-real-time mode of operation of said system;

receiving information respective of an image flip value of said image, from a user interface, in a real-time mode of operation of said system; and

determining an image flip correction model corresponding to said image flip value, according to said logical relationship, in said real-time mode of operation of said system.

27. The method according to claim 1, further comprising:

determining a plurality of image rotation correction models corresponding to respective ones of a plurality of image rotation values of another image of said body region of interest, in said non-real-time mode of operation of said system;

constructing a logical relationship between said image rotation correction models and said respective image rotation values, in said non-real-time mode of operation of said system;

receiving information respective of an image rotation value of said image, from said image detector, in a real-time mode of operation of said system; and

determining an image rotation correction model corresponding to said image rotation value, according to said logical relationship, in said real-time mode of operation of said system.

28. The method according to claim 1, further comprising:

determining a plurality of image flip correction models corresponding to respective ones of a plurality of image flip values of another image of said body region of interest, in said non-real-time mode of operation of said system;

constructing a logical relationship between said image flip correction models and said respective image flip values, in said non-real-time mode of operation of said system;

receiving information respective of an image flip value of said image, from said image detector, in a real-time mode of operation of said system; and

determining an image flip correction model corresponding to said image flip value, according to said logical relationship, in said real-time mode of operation of said system.

29. The method according to claim 1, wherein each of said representation and said image is real-time.

30. The method according to claim 1, wherein said representation is real-time and said image is acquired previously.

31. The method according to claim 1, wherein said representation is acquired previously and said image is real-time.

32. The method according to claim 1, wherein each of said representation and said image is acquired previously.

33. System for displaying a representation of the tip of a medical device located within a body region of interest (120) of a patient (122), on an image of the body region of interest, the image being acquired by an image detector (132) of a moving imager (102) at one or more physical zoom settings of said image detector (132) respective of said image, and at a selected image detector region of interest setting of said image detector (132), the system comprising:

at least one magnetic field generator (118) firmly coupled with a moving portion of said moving imager (102), said at least one magnetic field generator (118) producing a magnetic field at said body region of interest;

a medical device medical positioning system (MPS) sensor (112, 114, 116) coupled with said tip of said medical device, said medical device MPS sensor (112, 114, 116) detecting said magnetic field;

an MPS (104) coupled with said at least one magnetic field generator (118) and with said medical device MPS sensor (112, 114, 116), said at least one magnetic field generator (118) being associated with an MPS coordinate system respective of said MPS (102), said MPS determining MPS coordinates of said medical device MPS sensor (112, 114, 116), according to an output of said medical device MPS sensor (112, 114, 116); and

a processor (108) coupled with said MPS (102), said processor (108) determining two-dimensional coordinates of said tip of said medical device located within said body region of interest, according to a physical zoom setting of said image detector (132) respective of said image, according to a set of intrinsic and extrinsic parameters respective of said image detector (132), said set of intrinsic and extrinsic parameters being determined at a selected physical zoom setting, according to sensor image coordinates of each of the MPS sensor images in a two-dimensional optical coordinate system respective of said image detector (132) and according to non-real time MPS coordinates of said MPS sensor, in an MPS coordinate system respective of said MPS, wherein each set of intrinsic parameters comprising optical characteristics of said image detector and each set of extrinsic parameters comprising rotation and translation parameters of the MPS coordinate system relative to the two-dimensional coordinate system, according to said selected image detector region of interest setting of said image detector, and according to said MPS coordinates of said medical device MPS sensor, said processor superimposing a representation of said tip of said medical device, on said image, according to said two-dimensional coordinates, wherein the processor (108) is **characterised in that** it is adapted for

determining said set of intrinsic and extrinsic parameters with respect to a plurality of physical zoom settings of said image detector, by interpolating between intrinsic and extrinsic parameters associated with two adjacent ones of said physical zoom settings or by extrapolating beyond intrinsic and extrinsic parameters associated with two adjacent ones of said physical zoom settings.

34. The system according to claim 33, further comprising a user interface coupled with said processor, said user interface receiving an input from a user.

35. The system according to claim 34, wherein said input is selected from the list consisting of:

   rotation angle of said image;
   flip type of said image; and
   viewing position value of said image detector.

36. The system according to claim 33, further comprising a display coupled with said processor, said display displaying a superposition of said representation on said image.

37. The system according to claim 33, wherein said selected image detector region of interest setting is one of one or more image detector region of interest settings corresponding to one or more regions of interest, the system further comprising a full span fiducial screen coupled with said image detector, in front of said image detector, in an off-line mode of operation of said system, said full span fiducial screen including a plurality of fiducials comprising at least one group of complementary fiducials, wherein every fiducial in a group is complementary to the rest of said fiducials in said group, said processor determining a scale function between different image detector regions of interest, according to fiducial image coordinates of said fiducials, in a plurality of fiducial images acquired by said image detector, from said full span fiducial screen, at said different image detector regions of interest, and at at least one physical zoom setting of said image detector, and according to actual coordinates of said fiducials.

38. The system according to claim 33, further comprising a peripheral fiducial screen located in a field of view of said image detector, in a non-real-time mode of operation of said system, said peripheral fiducial screen including a plurality of peripheral fiducials comprising at least one group of complementary fiducials, wherein every peripheral fiducial in a group is complementary to the rest of said peripheral fiducials in said group, said image detector acquiring at least one reference image of said body region of interest, in said non-real-time mode of operation of said system, at a reference position of said moving imager, at each physical zoom setting of said image detector, and at each image detector region of interest setting of said image detector, each of said at least one reference image including a plurality of peripheral fiducial images of said peripheral fiducials, at a periphery of said at least one reference image, wherein said processor determines a viewing position transformation model respective of a selected viewing position of said image detector, in a real-time mode of operation of said system, according to a first set of coordinates of said peripheral fiducials in said at least one reference image, and according to a second set of coordinates of said peripheral fiducials, in said image.

39. The system according to claim 38, further comprising a database coupled with said processor, wherein said processor determines a plurality of image rotation correction models, respective of respective ones of a plurality of image rotation values of said at least one reference image, according to said first set of coordinates, in said non-real-time mode of operation of said system,
wherein said processor constructs a logical relationship between said image rotation correction models, and said image rotation values, in said non-real-time mode of operation of said system,
wherein said processor stores said logical relationship in said database, wherein said processor determines an image rotation correction model corresponding to a selected image rotation value of said image, in said real-time mode of operation of said system, by incorporating said second set of coordinates in said logical relationship, and wherein said processor determines said two-dimensional coordinates of said tip of said medical device, furthermore according to said image rotation correction model.

40. The system according to claim 38, further comprising a database coupled with said processor, wherein said processor determines a plurality of image flip correction models, respective of respective ones of a plurality of image flip values of said at least one reference image, according to said first set of coordinates, in said non-real-time mode of operation of said system,
wherein said processor constructs a logical relationship between said image flip correction models, and said image flip values, in said non-real-time mode of operation of said system,
wherein said processor stores said logical relationship in said database,
wherein said processor determines an image flip correction model corresponding to a selected image flip value of said image, in said real-time mode of operation of said system, by incorporating said second set of coordinates in said logical relationship, and
wherein said processor determines said two-dimensional coordinates of said tip of said medical device, furthermore according to said image flip correction model.

41. The system according to claim 33, further comprising:

a peripheral fiducial screen located in a field of view of said image detector, in a non-real-time mode of operation of said system, said peripheral fiducial screen including a plurality of peripheral fiducials comprising at least one group of complementary fiducials, wherein every peripheral fiducial in a group is complementary to the rest of said peripheral fiducials in said group, said image detector acquiring at least one reference image of said body region of interest, in said non-real-time mode of operation of said system, at a reference position of said moving imager, at each physical zoom setting of said image detector, and at each image detector region of interest setting of said image detector, each of said at least one reference image including a plurality of peripheral fiducial images of said peripheral fiducials, at a periphery of said at least one reference image;
a database coupled with said processor, wherein said processor determines a plurality of viewing position transformation models corresponding to respective ones of a plurality of viewing position values of said image detector, in a non-real-time mode of operation of said system, according to fiducial image coordinates of said peripheral fiducials in respective ones of said at least one reference image, and according to actual coordinates of said peripheral fiducials;
wherein said processor constructs a first logical relationship between said viewing position transformation models, and said respective viewing position values, in said non-real-time mode of operation of said system,
wherein said processor receives information respective of a viewing position value of said image detector, from a user interface, in a real-time mode of operation of said system; and
wherein said processor determines a viewing position transformation model corresponding to said viewing position value, according to said first logical relationship, in said real-time mode of operation of said system.

42. The system according to claim 33, further comprising a position detector coupled with said moving imager and with said processor, said processor determining a position of said moving imager according to an output of said position detector.

43. The system according to claim 33, further comprising a reference MPS sensor fixed at a reference location, said reference MPS sensor being coupled with said MPS, said MPS determining a position of said moving imager according to an output of said reference MPS sensor.

44. The system according to claim 33, further comprising an image detector MPS sensor coupled with said image detector and with said MPS, said moving imager including an emitter located on an opposite side of said patient relative to the location of said image detector, said emitter emitting radiation toward said image detector along a radiation axis, said MPS determining a position of said image detector along said radiation axis, according to an output of said image detector MPS sensor.

45. The system according to claim 33, further comprising a patient body MPS sensor firmly coupled with the body of said patient and with said MPS, said MPS determining a viewing position value of said image detector relative to said body, according to an output of said patient body MPS sensor, said processor compensating for the movements of said patient, and of said moving imager, while said processor processes data respective of images which said image detector detects.

46. The system according to claim 33, wherein each of said representation and said image is real-time.

47. The system according to claim 33, wherein said representation is real-time and said image is acquired previously.

48. The system according to claim 33, wherein said representation is acquired previously and said image is real-time.

49. The system according to claim 33, wherein each of said representation and said image is acquired previously.

50. The system according to claim 33, wherein said image detector is an image intensifier.

51. The system according to claim 33, wherein said image detector is a flat detector.

52. The system according to claim 33, wherein said magnetic field generators are coupled with said image detector.

53. The system according to claim 33, wherein said moving imager includes an emitter located on an opposite side of said patient relative to the location of said image detector, said emitter emitting radiation toward said image detector, said magnetic field generators being coupled with said emitter.

54. The system according to claim 33, wherein said moving imager is a computer assisted tomography (CAT) machine, said CAT including a CAT image detector and a CAT emitter, said CAT emitter being located on an opposite side of said patient relative to the location of said CAT image detector, said CAT emitter emitting radiation toward said CAT image detector, said magnetic field generators being coupled with said CAT image detector.

55. The system according to claim 33, wherein said moving imager operates according to a principle selected from the list consisting of:

X-rays;
nuclear magnetic resonance;
elementary particle emission;
and thermography.

56. The system according to claim 33, wherein said medical device is selected from the list consisting of:

balloon catheter;
stent carrying catheter;
medical substance dispensing catheter;
suturing catheter;
guidewire;
ablation unit;
brachytherapy unit;
intravascular ultrasound catheter;
lead of a cardiac rhythm treatment device;
lead of an intra-body cardiac defibrillator device;
guiding device of a lead of a cardiac rhythm treatment device; guiding device of a lead of an intra-body cardiac device; valve treatment catheter;
valve implantation catheter;
intra-body ultrasound catheter;

intra-body computer tomography catheter;
therapeutic needle;
diagnostic needle;
gastroenterology device;
orthopedic device;
neurosurgical device;
intra-vascular flow measurement device;
intra-vascular pressure measurement device;
intra-vascular optical coherence tomography device;
intra-vascular near infrared spectroscopy device;
intra-vascular infrared device; and
otorhinolaryngology precision surgery device.

**Patentansprüche**

1. Verfahren zur Anzeige einer Darstellung der Spitze einer medizinischen Vorrichtung, die sich in einer Körperregion von Interesse (120) des Körpers eines Patienten (122) befindet, auf einem Bild der Körperregion, die von Interesse ist, wobei das Bild durch einen Bilddetektor (132) eines sich bewegenden Bildgebers (102) bei einer ausgewählten physikalischen Zoomeinstellung erfasst wird, wobei das Verfahren aufweist:

Erfassen mindestens eines MPS (Medical Positioning System)-Sensorbilds eines MPS-Sensors durch den Bilddetektor (132), bei einer oder bei mehreren physikalischen Zoomeinstellungen des Bilddetektors (132) entsprechend dem Bild, und bei einer ausgewählten Bilddetektorregioneinstellung des Bilddetektors (132), wobei der MPS-Sensor einem MPS (104) zugeordnet ist, wobei der mindestens eine MPS-Sensor (112, 114, 116) auf ein elektromagnetisches Feld antwortet, das von einer Mehrzahl von elektromagnetischen Feldgeneratoren (118) erzeugt wird, die fest mit einem sich bewegenden Bereich des sich bewegenden Bildgebers (102) gekoppelt sind;
Bestimmen eines Satzes von intrinsischen und extrinsischen Parametern bei der ausgewählten physikalischen Zoomeinstellung gemäß Sensorbildkoordinaten jedes MPS-Sensorbilds, in einem zweidimensionalen optischen Koordinatensystem entsprechend dem Bilddetektor (122), und gemäß Nicht-Echtzeit-MPS-Koordinaten des MPS-Sensors (112, 114, 116) in einem MPS-Koordinatensystem entsprechend dem MPS, wobei jeder Satz von intrinsischen Parametern optische Eigenschaften des Bilddetektors aufweist, und jeder Satz von extrinsischen Parametern Drehungs- und Translationsparameter des MPS-Koordinatensystems relativ zu dem optischen Koordinatensystem aufweist;
Bestimmen von zweidimensionalen Koordinaten der Spitze der medizinischen Vorrichtung gemäß der ausgewählten physikalischen Zoomeinstellung gemäß dem Satz von intrinsischen und extrinsischen Parametern gemäß der ausgewählten Bilddetektorregioneinstellung, und gemäß Echtzeit-MPS-Koordinaten eines MPS-Sensors, der sich an der Spitze der medizinischen Vorrichtung befindet;
Überlagern der Darstellung der Spitze der medizinischen Vorrichtung auf das Bild der Körperregion, die von Interesse ist, gemäß den zweidimensionalen Koordinaten; und
Anzeigen der Darstellung der Spitze der medizinischen Vorrichtung, die auf das Bild der Körperregion, die von Interesse ist, überlagert ist, wobei das Verfahren **dadurch gekennzeichnet ist, dass**
der Schritt des Bestimmens des Satzes von intrinsischen und extrinsischen Parametern durchgeführt wird entsprechend einer Mehrzahl von physikalischen Zoomeinstellungen des Bilddetektors, durch Interpolieren zwischen intrinsischen und extrinsischen Parametern, die zu zwei benachbarten Einstellungen der physikalischen Zoomeinstellungen gehören, oder durch Extrapolieren jenseits der intrinsischen und extrinsischen Parameter, die zu zwei benachbarten Einstellungen der physikalischen Einstellungen gehören.

2. Verfahren nach Anspruch 1, ferner mit einem Schritt des Entfernens eines vollflächigen Referenzbildschirms aus einem Betrachtungsfeld des Bilddetektors, wobei der vollflächige Referenzbildschirm in dem Betrachtungsfeld, in einem Offline-Modus des Betriebs eines Systems platziert worden ist, das gemäß dem Verfahren arbeitet, wobei der vollflächige Referenzbildschirm eine Mehrzahl von Referenzen aufweist, mit mindestens einer Gruppe von komplementären Referenzen, wobei jede Referenz in der Gruppe komplementär zu dem Rest der Referenzen in der Gruppe ist.

3. Verfahren nach Anspruch 2, bei dem die ausgewählte Bilddetektorregioneinstellung eine oder mehrere Regioneinstellungen aufweist, die einer oder mehreren Regionen von Interesse entspricht, ferner mit einem Schritt zum Bestimmen einer Skalierungsfunktion zwischen unterschiedlichen Bilddetektorregionen von Interesse, gemäß Referenzbildkoordinaten der mindestens einen Gruppe von komplementären Referenzen in einer Mehrzahl von Referenzbildern, die durch den Bilddetektor bei den unterschiedlichen Bilddetektorregionen, die von Interesse sind und bei der ausgewählten physikalischen Zoomeinstellung erfasst werden, und gemäß den tatsächlichen Koordinaten der komplementären Referenzen.

4. Verfahren nach Anspruch 4, bei dem der Vorgang des Bestimmens der zweidimensionalen Koordinaten durchgeführt wird gemäß der Skalierungsfunktion.

5. Verfahren nach Anspruch 3, ferner mit einem Schritt zum Erfassen der Referenzbilder durch den Bilddetektor (132).

6. Verfahren nach Anspruch 5, ferner mit einem Schritt zum Platzieren des vollflächigen Referenzbildschirms in dem Betrachtungsfeld.

7. Verfahren nach Anspruch 1, bei dem das mindestens eine MPS-Sensorbild ein einzelnes MPS-Sensorbild einer Mehrzahl von MPS-Sensoren aufweist.

8. Verfahren nach Anspruch 1, bei dem die ausgewählte Bilddetektorregion, die von Interesse ist, die größte Bilddetektorregion von Interesse ist, von einer Mehrzahl von Bilddetektorregionen, die von Interesse sind.

9. Verfahren nach Anspruch 1, ferner mit einem Schritt zum:

   festen Anbringen eines peripheren Referenzbildschirms an dem Bilddetektor, vor dem Bilddetektor, in einem Nicht-Echtzeit-Betriebsmodus eines Systems, das gemäß dem Verfahren arbeitet, wobei der periphere Referenzbildschirm eine Mehrzahl von peripheren Referenzen aufweist, mit mindestens einer Gruppe von komplementären Referenzen, wobei jede Referenz in einer Gruppe komplementär ist zu dem Rest der peripheren Referenzen in dieser Gruppe;
   Erfassen von mindestens einem Referenzbild der Körperregion, die von Interesse ist, durch den Bilddetektor in dem Nicht-Echtzeit-Betriebsmodus des Systems, bei einer Referenzposition des sich bewegenden Bildgebers, bei jeder physikalischen Zoomeinstellung des Bilddetektors, und bei jeder Bilddetektorregioneinstellung des Bilddetektors, wobei jedes der mindestens einen Referenzbilder eine Mehrzahl von peripheren Referenzbildern von peripheren Referenzen aufweist, bei einer Peripherie des mindestens einen Referenzbilds; und
   Bestimmen eines Betrachtungspositionstransformationsmodells entsprechend einer Betrachtungsposition des Bilddetektors, in einem Echtzeit-Betriebsmodus des Systems gemäß einem ersten Satz von Koordinaten der peripheren Referenzen in dem mindestens einen Referenzbild, und gemäß einem zweiten Satz von Koordinaten der peripheren Referenzen in einem Echtzeitbild der Körperregion, die von Interesse ist, die von dem Bilddetektor erfasst wird.

10. Verfahren nach Anspruch 9, bei dem der Schritt des Bestimmens der Koordinaten der Spitze der medizinischen Vorrichtung ferner durchgeführt wird gemäß dem Betrachtungspositionstransformationsmodell.

11. Verfahren nach Anspruch 9, ferner mit:

    Bestimmen eines Satzes von Bildrotationskorrekturmodellen bezüglich des ersten Satzes von Koordinaten in jedem von dem mindestens einen Referenzbild in einem Nicht-Echtzeit-Betriebsmodus des Systems;
    Konstruieren einer logischen Beziehung zwischen jedem Bildrotationskorrekturmodell des Satzes von Bildrotationskorrekturmodellen und dem entsprechenden ersten Satz von Koordinaten in dem Nicht-Echtzeit-Betriebsmodus des Systems;
    Bestimmen eines Bildrotationskorrekturmodells entsprechend dem zweiten Satz von Koordinaten gemäß der logischen Beziehung in einem Echtzeit-Betriebsmodus des Systems; und
    Durchführen des Vorgangs des Bestimmens der zweidimensionalen optischen Koordinaten der Spitze der medizinischen Vorrichtung ferner gemäß dem Bildrotationkorrekturmodell.

12. Verfahren nach Anspruch 9, ferner mit:

    Bestimmen eines Satzes von Bildwechselkorrekturmodellen entsprechend dem ersten Satz von Koordinaten in jedem von dem mindestens einen Referenzbild in einem Nicht-Echtzeit-Betriebsmodus des Systems;
    Konstruieren einer logischen Beziehung zwischen jedem Bildwechselkorrekturmodell des Satzes von Bildrotationskorrekturmodellen, und dem entsprechenden ersten Satz von Koordinaten in einem Nicht-Echtzeit-Betriebsmodus des Systems;
Bestimmen eines Bildwechselkorrekturmodells entsprechend dem zweiten Satz von Koordinaten gemäß der logischen Beziehung in einem Echtzeit-Betriebsmodus des Systems; und
Durchführen des Vorgangs des Bestimmens der zweidimensionalen Koordinaten der Spitze der medizinischen Vorrichtung ferner gemäß dem Bildwechselkorrekturmodell.

**13.** Verfahren nach Anspruch 1, ferner mit:

Bestimmen einer Mehrzahl von Betrachtungspositionsverzerrungsmodellen entsprechend jeweiligen Werten einer Mehrzahl von Betrachtungspositionswerten des Bilddetektors in einem Nicht-Echtzeit-Betriebsmodus des Systems, das gemäß dem Verfahren arbeitet,
Konstruieren einer ersten logischen Beziehung zwischen den Betrachtungspositionsverzerrungsmodellen und den jeweiligen Betrachtungspositionswerten in dem Nicht-Echtzeit-Betriebsmodus des Systems;
Empfangen von Information bezüglich eines Betrachtungspositionswerts des Bilddetektors von einer Benutzerschnittstelle in einem Echtzeit-Betriebsmodus des Systems; und
Bestimmen eines Betrachtungspositionsverzerrungsmodells entsprechend dem Betrachtungspositionswert gemäß der ersten logischen Beziehung in dem Echtzeit-Betriebsmodus des Systems.

**14.** Verfahren nach Anspruch 13, bei dem der Schritt des Bestimmens der zweidimensionalen Koordinaten der Spitze der medizinischen Vorrichtung ferner durchgeführt wird gemäß dem Betrachtungspositionstransformationsmodell.

**15.** Verfahren nach Anspruch 13, ferner mit:

Bestimmen einer Mehrzahl von Bildrotationskorrekturmodellen entsprechend jeweiligen Werten einer Mehrzahl von Bildrotationswerten eines anderen Bilds der Körperregion von Interesse in dem Nicht-Echtzeit-Betriebsmodus des Systems,
Konstruieren einer zweiten logischen Beziehung zwischen den Bildrotationskorrekturmodellen und den jeweiligen Bildrotationswerten in dem Nicht-Echtzeit-Betriebsmodus des Systems;
Empfangen einer Information entsprechend eines Bildrotationswerts des Bilds von einer Benutzerschnittstelle in einem Echtzeit-Betriebsmodus des Systems; und
Bestimmen eines Bildrotationskorrekturmodells entsprechend dem Bildrotationswert gemäß der zweiten logischen Beziehung in dem Echtzeit-Betriebsmodus des Systems.

**16.** Verfahren nach Anspruch 13, ferner mit:

Bestimmen einer Mehrzahl von Bildwechselkorrekturmodellen entsprechend jeweiligen Werten einer Mehrzahl von Bildwechselwerten eines anderen Bilds der Körperregion von Interesse in dem Nicht-Echtzeit-Betriebsmodus des Systems,
Konstruieren einer zweiten logischen Beziehung zwischen den Bildwechselkorrekturmodellen und den jeweiligen Bildwechselwerten in dem Nicht-Echtzeit-Betriebsmodus des Systems;
Empfangen einer Information entsprechend eines Bildwechselwerts des Bilds von einer Benutzerschnittstelle in einem Echtzeit-Betriebsmodus des Systems; und
Bestimmen eines Bildwechselkorrekturmodells entsprechend dem Bildwechselwert gemäß der zweiten logischen Beziehung in dem Echtzeit-Betriebsmodus des Systems.

**17.** Verfahren nach Anspruch 1, ferner mit:

festes Anbringen eines peripheren Referenzbildschirms an dem Bilddetektor vor dem Bilddetektor, in einem Nicht-Echtzeit-Betriebsmodus eines Systems, das gemäß dem Verfahren arbeitet, wobei der periphere Referenzbildschirm eine Mehrzahl von peripheren Referenzen aufweist, die mindestens eine Gruppe von komplementären peripheren Referenzen aufweist, wobei jede periphere Referenz in einer Gruppe komplementär ist zu dem Rest der peripheren Referenzen in dieser Gruppe;
Erfassen von mindestens einem Referenzbild der Körperregion von Interesse durch den Bilddetektor in dem Nicht-Echtzeit-Betriebsmodus des Systems bei einer Referenzposition des sich bewegenden Bildgebers bei jeder physikalischen Zoomeinstellung des Bilddetektors, und bei jeder Bilddetektorregioneinstellung des Bilddetektors, wobei jedes von dem mindestens einen Referenzbild eine Mehrzahl von peripheren Referenzbildern der peripheren Referenzen aufweist bei einer Peripherie des mindestens einen Referenzbilds;

Bestimmen einer Mehrzahl von Betrachtungspositionstransformationsmodellen entsprechend jeweiligen Werten einer Mehrzahl von Betrachtungspositionswerten des Bilddetektors in einem Nicht-Echtzeit-Betriebsmodus des Systems, das gemäß Referenzbildkoordinaten der peripheren Referenzen in entsprechenden Bildern von dem mindestens einen Referenzbild, und gemäß tatsächlichen Koordinaten der peripheren Referenzen;
Konstruieren einer ersten logischen Beziehung zwischen den Betrachtungspositionstransformationsmodellen und den jeweiligen Betrachtungspositionswerten in dem Nicht-Echtzeit-Betriebsmodus des Systems;
Empfangen einer Information bezüglich eines Betrachtungspositionswerts des Bilddetektors von dem Bilddetektor in einem Echtzeit-Betriebsmodus des Systems; und
Bestimmen eines Betrachtungspositionstransformationsmodells entsprechend dem Betrachtungspositionswert gemäß der ersten logischen Beziehung in dem Echtzeit-Betriebsmodus des Systems.

18. Verfahren nach Anspruch 17, bei dem der Schritt des Bestimmens der zweidimensionalen optischen Koordinaten der Spitze der medizinischen Vorrichtung ferner durchgeführt wird gemäß dem Betrachtungspositionstransformationsmodell.

19. Verfahren nach Anspruch 17, ferner mit:

Bestimmen einer Mehrzahl von Bildrotationskorrekturmodellen entsprechend jeweiligen Werten einer Mehrzahl von Bildrotationswerten eines anderen Bilds der Körperregion von Interesse in dem Nicht-Echtzeit-Betriebsmodus des Systems;
Konstruieren einer zweiten logischen Beziehung zwischen den Bildrotationskorrekturmodellen und den jeweiligen Bildrotationswerten in dem Nicht-Echtzeit-Betriebsmodus des Systems;
Empfangen einer Information bezüglich eines Bildrotationswerts des Bilds von dem Bilddetektor in einem Echtzeit-Betriebsmodus des Systems; und
Bestimmen eines Bildrotationskorrekturmodells entsprechend dem Bildrotationswert gemäß der zweiten logischen Beziehung in dem Echtzeit-Betriebsmodus des Systems.

20. Verfahren nach Anspruch 17, ferner mit:

Bestimmen einer Mehrzahl von Bildwechselkorrekturmodellen entsprechend jeweiligen Werten einer Mehrzahl von Bildwechselwerten eines anderen Bilds der Körperregion von Interesse in dem Nicht-Echtzeit-Betriebsmodus des Systems;
Konstruieren einer zweiten logischen Beziehung zwischen den Bildwechselkorrekturmodellen und den jeweiligen Bildwechselwerten in dem Nicht-Echtzeit-Betriebsmodus des Systems;
Empfangen einer Information entsprechend eines Bildwechselwerts des Bilds von dem Bilddetektor in einem Echtzeit-Betriebsmodus des Systems; und
Bestimmen eines Bildwechselkorrekturmodells entsprechend dem Bildwechselwert gemäß der zweiten logischen Beziehung in dem Echtzeit-Betriebsmodus des Systems.

21. Verfahren nach Anspruch 1, ferner mit:

festes Anbringen eines peripheren Referenzbildschirms an dem Bilddetektor, vor dem Bilddetektor in einem Nicht-Echtzeit-Betriebsmodus eines Systems, das gemäß dem Verfahren arbeitet, wobei der periphere Referenzbildschirm eine Mehrzahl von peripheren Referenzen aufweist, die mindestens einer Gruppe von komplementären peripheren Referenzen entsprechen, wobei jede periphere Referenz in einer Gruppe komplementär ist zu dem Rest der peripheren Referenzen in dieser Gruppe;
Erfassen von mindestens einem Referenzbild der Körperregion von Interesse durch den Bilddetektor in einem Nicht-Echtzeit-Betriebsmodus des Systems bei einer Referenzposition des sich bewegenden Bildgebers bei jeder physikalischen Zoomeinstellung des Bilddetektors, und bei jeder Bilddetektorregion von Interesse des Bilddetektors, wobei jedes von dem mindestens einen Referenzbild eine Mehrzahl von peripheren Referenzbildern der peripheren Referenzen aufweist bei einer Peripherie des mindestens einen Referenzbilds; und
Bestimmen eines Bildrotationskorrekturmodells entsprechend einem Bildrotationswert des Bilds in einem Echtzeit-Betriebsmodus des Systems gemäß einem ersten Satz von Koordinaten der peripheren Referenzen in dem mindestens einen Referenzbild, und gemäß einem zweiten Satz von Koordinaten der peripheren Referenzen in dem Bild.

22. Verfahren nach Anspruch 21, bei dem der Schritt des Bestimmens der zweidimensionalen Koordinaten der Spitze der medizinischen Vorrichtung ferner durchgeführt wird gemäß dem Bildrotationskorrekturmodell.

**23.** Verfahren nach Anspruch 1, ferner mit:

festem Anbringen eines peripheren Referenzbildschirms an dem Bilddetektor vor dem Bilddetektor in einem Nicht-Echtzeit-Betriebsmodus eines Systems, das gemäß dem Verfahren arbeitet, wobei der periphere Referenzbildschirm eine Mehrzahl von peripheren Referenzen aufweist, die mindestens eine Gruppe von komplementären Referenzen aufweist, wobei jede periphere Referenz in einer Gruppe komplementär ist zu dem Rest der peripheren Referenzen in dieser Gruppe;
Erfassen von mindestens einem Referenzbild der Körperregion von Interesse durch den Bilddetektor in einem Nicht-Echtzeit-Betriebsmodus des Systems bei einer Referenzposition des sich bewegenden Bildgebers bei jeder physikalischen Zoomeinstellung des Bilddetektors, und bei jeder Bilddetektorregion von Interesse des Bilddetektors, wobei jedes von dem mindestens einen Referenzbild eine Mehrzahl von peripheren Referenzbildern der peripheren Referenzen aufweist bei einer Peripherie des mindestens einen Referenzbilds; und
Bestimmen eines Bildwechselkorrekturmodells entsprechend einem Bildrotationswert des Bilds in einem Echtzeit-Betriebsmodus des Systems gemäß einem ersten Satz von Koordinaten der peripheren Referenzen in dem mindestens einen Referenzbild, und gemäß einem zweiten Satz von Koordinaten der peripheren Referenzen in diesem Bild.

**24.** Verfahren nach Anspruch 23, bei dem der Schritt des Bestimmens der zweidimensionalen Koordinaten der Spitze der medizinischen Vorrichtung ferner durchgeführt wird gemäß dem Bildwechselkorrekturmodell.

**25.** Verfahren nach Anspruch 1, ferner mit:

Bestimmen einer Mehrzahl von Bildrotationskorrekturmodellen entsprechend jeweiligen Werten von einer Mehrzahl von Bildrotationswerten eines anderen Bilds der Körperregion von Interesse in einem Nicht-Echtzeit-Betriebsmodus des Systems;
Konstruieren einer logischen Beziehung zwischen den Bildrotationskorrekturmodellen und den jeweiligen Bildrotationswerten in dem Nicht-Echtzeit-Betriebsmodus des Systems;
Empfangen von Information bezüglich eines Bildrotationswerts des Bilds von einer Benutzerschnittstelle in einem Echtzeit-Betriebsmodus des Systems; und
Bestimmen eines Bildrotationskorrekturmodells entsprechend dem Bildrotationswert gemäß der logischen Beziehung in dem Echtzeit-Betriebsmodus des Systems.

**26.** Verfahren nach Anspruch 1, ferner mit:

Bestimmen einer Mehrzahl von Bildwechselkorrekturmodellen entsprechend jeweiligen Werten von einer Mehrzahl von Bildwechselwerten eines anderen Bilds der Körperregion von Interesse in dem Nicht-Echtzeit-Betriebsmodus des Systems;
Konstruieren einer logischen Beziehung zwischen den Bildwechselkorrekturmodellen und den jeweiligen Bildwechselwerten in dem Nicht-Echtzeit-Betriebsmodus des Systems;
Empfangen einer Information bezüglich eines Bildwechselwerts des Bilds von einer Benutzerschnittstelle in einem Echtzeit-Betriebsmodus des Systems; und
Bestimmen eines Bildwechselkorrekturmodells entsprechend dem Bildwechselwert gemäß der logischen Beziehung in dem Echtzeit-Betriebsmodus des Systems.

**27.** Verfahren nach Anspruch 1, ferner mit:

Bestimmen einer Mehrzahl von Bildrotationskorrekturmodellen entsprechend jeweiligen Werten von einer Mehrzahl von Bildrotationswerten eines anderen Bilds der Körperregion von Interesse in dem Nicht-Echtzeit-Betriebsmodus des Systems;
Konstruieren einer logischen Beziehung zwischen den Bildrotationskorrekturmodellen und den jeweiligen Bildrotationswerten in dem Nicht-Echtzeit-Betriebsmodus des Systems;
Empfangen einer Information entsprechend einem Bildrotationswert des Bilds von dem Bilddetektor in einem Echtzeit-Betriebsmodus des Systems; und
Bestimmen eines Bildrotationskorrekturmodells entsprechend dem Bildrotationswert gemäß der logischen Beziehung in dem Echtzeit-Betriebsmodus des Systems.

**28.** Verfahren nach Anspruch 1, ferner mit:

Bestimmen einer Mehrzahl von Bildwechselkorrekturmodellen entsprechend jeweiligen Werten von einer Mehrzahl von Bildwechselwerten eines anderen Bilds der Körperregion von Interesse in dem Nicht-Echtzeit-Betriebsmodus des Systems;

Konstruieren einer logischen Beziehung zwischen den Bildwechselkorrekturmodellen und den jeweiligen Bildwechselwerten in dem Nicht-Echtzeit-Betriebsmodus des Systems;

Empfangen einer Information bezüglich eines Bildwechselwerts des Bilds von dem Bilddetektor in einem Echtzeit-Betriebsmodus des Systems; und

Bestimmen eines Bildwechselkorrekturmodells entsprechend dem Bildwechselwert gemäß der logischen Beziehung in dem Echtzeit-Betriebsmodus des Systems.

29. Verfahren nach Anspruch 1, bei dem die Darstellung und das Bild in Echtzeit ist.

30. Verfahren nach Anspruch 1, bei dem die Darstellung in Echtzeit und das Bild zuvor erfasst wird.

31. Verfahren nach Anspruch 1, bei dem die Darstellung zuvor erfasst wird und das Bild in Echtzeit ist.

32. Verfahren nach Anspruch 1, bei dem die Darstellung und das Bild zuvor erfasst werden.

33. System zur Anzeige einer Darstellung der Spitze einer medizinischen Vorrichtung, die sich in einer Körperregion von Interesse (120) eines Patienten (122) befindet, auf einem Bild der Körperregion von Interesse, wobei das Bild durch einen Bilddetektor (132) eines sich bewegenden Bildgebers (102) bei einer oder mehreren physikalischen Zoomeinstellungen des Bilddetektors (132) entsprechend dem Bild erfasst werden, und bei einer ausgewählten Bilddetektorregioneinstellung des Bilddetektors (132), wobei das System aufweist:

mindestens einen Magnetfeldgenerator (118), der fest mit einem sich bewegenden Bereich des sich bewegenden Bildgebers (102) gekoppelt ist, wobei der mindestens eine Magnetfeldgenerator (118) ein Magnetfeld an einer Körperregion von Interesse erzeugt;

einen medizinischen Vorrichtungs-MPS (Medical Positioning Sensor)-Sensor (112, 114, 116), der mit der Spitze der medizinischen Vorrichtung gekoppelt ist, wobei der medizinische Vorrichtungs-MPS-Sensor (112, 114, 116) das Magnetfeld detektiert;

ein MPS (104), das mit dem mindestens einen Magnetfeldgenerator (118) und mit dem medizinischen Vorrichtungs-MPS-Sensor (112, 114, 116) gekoppelt ist, wobei der mindestens eine Magnetfeldgenerator (118) zu einem MPS-Koordinatensystem entsprechend dem MPS (102) gehört, wobei das MPS MPS-Koordinaten des medizinischen Vorrichtungs-MPS-Sensors (112, 114, 116) bestimmt gemäß einer Ausgabe des medizinischen Vorrichtungs-MPS-Sensors (112, 114, 116); und

einen Prozessor (108), der mit dem MPS (102) gekoppelt ist, wobei der Prozessor (108) zweidimensionale Koordinaten der Spitze der medizinischen Vorrichtung bestimmt, die sich in der Körperregion von Interesse befindet, gemäß einer physikalischen Zoomeinstellung des Bilddetektors (132) entsprechend dem Bild, gemäß einem Satz von intrinsischen und extrinsischen Parametern entsprechend dem Bilddetektor (132), wobei der Satz von intrinsischen und extrinsischen Parametern bei einer ausgewählten physikalischen Zoomeinstellung bestimmt wird, gemäß den Sensorbildkoordinaten von jedem der MPS-Sensorbilder in einem zweidimensionalen optischen Koordinatensystem entsprechend dem Bilddetektor (132) und gemäß Nicht-Echtzeit-MPS-Koordinaten des MPS-Sensors in einem MPS-Koordinatensystem entsprechend dem MPS, wobei jeder Satz von intrinsischen Parametern optische Eigenschaften des Bilddetektors aufweist und jeder Satz von extrinsischen Parametern Rotations- und Translationsparameter des MPS-Koordinatensystems bezüglich des zweidimensionalen Koordinatensystems aufweist, gemäß der ausgewählten Bilddetektorregioneinstellung des Bilddetektors, gemäß den MPS-Koordinaten des medizinischen Vorrichtungs-MPS-Sensors, wobei der Prozessor eine Darstellung der Spitze der medizinischen Vorrichtung auf das Bild überlagert gemäß den zweidimensionalen Koordinaten, wobei der Prozessor (108) **dadurch gekennzeichnet ist, dass** er angepasst ist zum

Bestimmen des Satzes von intrinsischen und extrinsischen Parametern bezüglich einer Mehrzahl von physikalischen Zoomeinstellungen des Bilddetektors durch Interpolieren zwischen intrinsischen und extrinsischen Parametern, die zu zwei benachbarten Einstellungen der physikalischen Zoomeinstellungen gehören, oder durch Extrapolieren jenseits der intrinsischen und extrinsischen Parameter, die zu zwei benachbarten Einstellungen der physikalischen Zoomeinstellungen gehören.

34. System nach Anspruch 33, ferner mit einer Benutzerschnittstelle, die mit dem Prozessor gekoppelt ist, wobei die Benutzerschnittstelle eine Eingabe von einem Benutzer erhält.

**35.** System nach Anspruch 34, bei dem die Eingabe ausgewählt ist aus einer Liste, die aufweist:

> Rotationswinkel des Bilds;
> Wechseltyp des Bilds; und
> Betrachtungspositionswert des Bilddetektors.

**36.** System nach Anspruch 33, ferner mit einer Anzeige, die mit dem Prozessor gekoppelt ist, wobei die Anzeige eine Überlagerung der Darstellung auf dem Bild anzeigt.

**37.** System nach Anspruch 33, bei dem die ausgewählte Bilddetektorregioneinstellung eine ist von einer oder von mehreren Bilddetektorregioneinstellungen, die eine oder mehreren Regionen von Interessen entsprechen, wobei das System ferner einen Vollflächenreferenzbildschirm aufweist, der mit dem Bilddetektor gekoppelt ist, vor dem Bilddetektor, in einem Offline-Betriebsmodus des Systems, wobei der Vollflächenreferenzbildschirm eine Mehrzahl von Referenzen aufweist, die mindestens eine Gruppe von komplementären Referenzen aufweisen, wobei jede Referenz in einer Gruppe komplementär ist zu dem Rest der Referenzen in dieser Gruppe, der Prozessor eine Skalierungsfunktion bestimmt zwischen unterschiedlichen Bilddetektorregionen von Interesse gemäß Referenzbildkoordinaten der Referenzen in einer Mehrzahl von Referenzbildern, die durch den Bilddetektor erfasst werden, von dem Vollflächenreferenzbildschirm bei den unterschiedlichen Bilddetektorregionen von Interesse, und bei mindestens einer physikalischen Zoomeinstellung des Bilddetektors, und gemäß tatsächlichen Koordinaten der Referenzen.

**38.** System nach Anspruch 33, ferner mit einem peripheren Referenzbildschirm, der sich in einem Betrachtungsfeld des Bilddetektors befindet, in einem Nicht-Echtzeit-Betriebsmodus des Systems, wobei der periphere Referenzbildschirm eine Mehrzahl von peripheren Referenzen aufweist, die mindestens eine Gruppe von komplementären Referenzen aufweisen, wobei jede periphere Referenz in einer Gruppe komplementär ist zu dem Rest der peripheren Referenzen in dieser Gruppe, der Bilddetektor mindestens ein Referenzbild der Körperregion von Interesse erfasst in dem Nicht-Echtzeit-Betriebsmodus des Systems bei einer Referenzposition des sich bewegenden Bildgebers bei jeder physikalischen Zoomeinstellung des Bilddetektors, und bei jeder Bilddetektorregioneinstellung des Bilddetektors, wobei jedes von dem mindestens einen Referenzbild eine Mehrzahl von peripheren Referenzbildern der peripheren Referenzen aufweist bei einer Peripherie des mindestens einen Referenzbilds, wobei der Prozessor ein Betrachtungspositionstransformationsmodell entsprechend einer ausgewählten Betrachtungsposition des Bilddetektors in einem Echtzeit-Betriebsmodus des Systems bestimmt gemäß einem ersten Satz von Koordinaten der peripheren Referenzen in dem mindestens einen Referenzbild, und gemäß einem zweiten Satz von Koordinaten der peripheren Referenzen in dem Bild.

**39.** System nach Anspruch 38, ferner mit einer Datenbank, die mit dem Prozessor gekoppelt ist, wobei der Prozessor eine Mehrzahl von Bildrotationskorrekturmodellen bestimmt entsprechend entsprechenden Werten von einer Mehrzahl von Bildrotationswerten des mindestens einen Referenzbilds gemäß dem ersten Satz von Koordinaten in einem Nicht-Echtzeit-Betriebsmodus des Systems, wobei der Prozessor eine logische Beziehung konstruiert zwischen den Bildrotationskorrekturmodellen und den Bildrotationswerten in einem Nicht-Echtzeit-Betriebsmodus des Systems, wobei der Prozessor die logische Beziehung in der Datenbank speichert, wobei der Prozessor ein Bildrotationskorrekturmodell bestimmt entsprechend einem ausgewählten Bildrotationswert des Bilds in dem Echtzeit-Betriebsmodus des Systems durch Inkorporation des zweiten Satzes von Koordinaten in die logische Beziehung, und wobei der Prozessor die zweidimensionalen Koordinaten der Spitze der medizinischen Vorrichtung ferner gemäß dem Bildrotationskorrekturmodell bestimmt.

**40.** System nach Anspruch 38, ferner mit einer Datenbank, die mit dem Prozessor gekoppelt ist, wobei der Prozessor eine Mehrzahl von Bildwechselkorrekturmodellen entsprechend entsprechenden Werten von einer Mehrzahl von Bildwechselwerten des mindestens einen Referenzbilds gemäß dem ersten Satz von Koordinaten in dem Nicht-Echtzeit-Betriebsmodus des Systems bestimmt, wobei der Prozessor eine logische Beziehung konstruiert zwischen den Bildwechselkorrekturmodellen und den Bildwechselwerten in dem Nicht-Echtzeit-Betriebsmodus des Systems, wobei der Prozessor die logische Beziehung in der Datenbank speichert, wobei der Prozessor ein Bildwechselkorrekturmodell entsprechend einem ausgewählten Bildwechselwert des Bilds bestimmt in dem Echtzeit-Betriebsmodus des Systems, in dem der zweite Satz von Koordinaten in die logische Beziehung eingebunden wird, und wobei der Prozessor die zweidimensionalen Koordinaten der Spitze der medizinischen Vorrichtung ferner gemäß dem Bildwechselkorrekturmodell bestimmt.

**41.** System nach Anspruch 33, ferner mit:

einem peripheren Referenzbildschirm, der sich in einem Betrachtungsfeld des Bilddetektors befindet, in einem Nicht-Echtzeit-Betriebsmodus des Systems, wobei der periphere Referenzbildschirm eine Mehrzahl von peripheren Referenzen aufweist, die mindestens eine Gruppe von komplementären Referenzen aufweisen, wobei jede periphere Referenz in einer Gruppe komplementär ist zu dem Rest der peripheren Referenzen in dieser Gruppe, der Bilddetektor mindestens ein Referenzbild der Körperregion von Interesse in dem Nicht-Echtzeit-Betriebsmodus des Systems bei einer Referenzposition des sich bewegenden Bildgebers bei jeder physikalischen Zoomeinstellung des Bilddetektors und bei jeder Bilddetektorregioneinstellung des Bilddetektors erfasst, wobei jedes von dem mindestens einen Referenzbild eine Mehrzahl von peripheren Referenzbildern der peripheren Referenzen aufweist bei einer Peripherie des mindestens einen Referenzbilds;

einer Datenbank, die mit dem Prozessor gekoppelt ist, wobei der Prozessor eine Mehrzahl von Betrachtungspositionstransformationsmodellen entsprechend entsprechenden Werten von einer Mehrzahl von Betrachtungspositionswerten des Bilddetektors bestimmt in einem Nicht-Echtzeit-Betriebsmodus des Systems gemäß Referenzbildkoordinaten der peripheren Referenzen in jeweiligen Bildern von dem mindestens einen Referenzbild, und gemäß tatsächlichen Koordinaten der peripheren Referenzen;

wobei der Prozessor eine erste logische Beziehung zwischen den Betrachtungspositionstransformationsmodellen und den jeweiligen Betrachtungspositionswerten in dem Nicht-Echtzeit-Betriebsmodus des Systems konstruiert,

wobei der Prozessor Information entsprechend eines Betrachtungspositionswerts des Bilddetektors von einer Benutzerschnittstelle in einem Echtzeit-Betriebsmodus des Systems erhält, und

wobei der Prozessor ein Betrachtungspositionstransformationsmodell entsprechend dem Betrachtungspositionswert gemäß der ersten logischen Beziehung in dem Echtzeit-Betriebsmodus des Systems bestimmt.

**42.** System nach Anspruch 33, ferner mit einem Positionsdetektor, der mit dem sich bewegenden Bildgeber und dem Prozessor gekoppelt ist, wobei der Prozessor eine Position des sich bewegenden Bildgebers gemäß einer Ausgabe des Positionsdetektors bestimmt.

**43.** System nach Anspruch 33, ferner mit einem Referenz-MPS-Sensor, der an einem Referenzort fixiert ist, wobei der Referenz-MPS-Sensor mit dem MPS gekoppelt ist, und das MPS eine Position des sich bewegenden Bildgebers gemäß einer Ausgabe des Referenz-MPS-Sensors bestimmt.

**44.** System nach Anspruch 33, ferner mit einem Bilddetektor-MPS-Sensor, der mit dem Bilddetektor und mit dem MPS gekoppelt ist, wobei der sich bewegende Bildgeber einen Emitter aufweist, der sich an einer gegenüberliegenden Seite des Patienten relativ zu dem Ort des Bilddetektors befindet, wobei der Emitter eine Strahlung in Richtung Bilddetektor entlang einer Strahlachse ausgibt, und das MPS eine Position des Bilddetektors entlang der Strahlungsachse gemäß einer Ausgabe des Bilddetektor-MPS-Sensors bestimmt.

**45.** System nach Anspruch 33, ferner mit einem Patientenkörper-MPS-Sensor, der fest mit dem Körper des Patienten und mit dem MPS gekoppelt ist, wobei das MPS einen Betrachtungspositionswert des Bilddetektors relativ zu dem Körper bestimmt gemäß einer Ausgabe des Patientenkörper-MPS-Sensors, der Prozessor Bewegungen des Patienten und des sich bewegenden Bildgebers kompensiert, während der Prozessor Daten entsprechend Bildern verarbeitet, die der Bilddetektor detektiert.

**46.** System nach Anspruch 33, bei dem die Darstellung und das Bild in Echtzeit sind.

**47.** System nach Anspruch 33, bei dem die Darstellung in Echtzeit ist und das Bild zuvor erfasst wird.

**48.** System nach Anspruch 33, bei dem die Darstellung zuvor erfasst wird und das Bild in Echtzeit ist.

**49.** System nach Anspruch 33, bei dem die Darstellung und das Bild zuvor erfasst werden.

**50.** System nach Anspruch 33, bei dem der Bilddetektor ein Bildintensivierer ist.

**51.** System nach Anspruch 33, bei dem der Bilddetektor ein flacher Detektor ist.

**52.** System nach Anspruch 33, bei dem Magnetfeldgeneratoren mit dem Bilddetektor gekoppelt sind.

53. System nach Anspruch 33, bei dem der sich bewegende Bildgeber einen Emitter aufweist, der sich an einer gegenüberliegenden Seite des Patienten relativ zu dem Ort des Bilddetektors befindet, wobei der Emitter eine Strahlung in Richtung Bilddetektor ausgibt, wobei die Magnetfeldgeneratoren mit dem Emitter gekoppelt sind.

54. System nach Anspruch 33, bei dem der sich bewegende Bildgeber eine computerunterstützte Tomografie (CAT)-Maschine ist, und CAT einen CAT-Bilddetektor und einen CAT-Emitter aufweist, wobei der CAT-Emitter sich auf einer gegenüberliegenden Seite des Patienten bezüglich des Orts des CAT-Bilddetektors befindet, und der CAT-Emitter eine Strahlung in Richtung CAT-Bilddetektor ausgibt, und die Magnetfeldgeneratoren mit dem CAT-Bilddetektor gekoppelt sind.

55. System nach Anspruch 33, bei dem der sich bewegende Bildgeber gemäß einem Prinzip arbeitet, das ausgewählt ist aus der Liste enthaltend:

> Röntgenstrahlen;
> nukleare Magnetresonanz;
> Elementarteilchenemission;
> und Thermografie.

56. System nach Anspruch 33, bei dem die medizinische Vorrichtung ausgewählt ist aus einer Liste enthaltend:

> einen Ballonkatheter;
> einen Stent-Tragekatheter;
> einen medizinischen Substanzabgabekatheter;
> einen Nähkatheter;
> einen Führungsdraht;
> eine Ablationseinheit;
> ein Brachytherapiegerät;
> ein intravaskulärer Ultraschallkatheter;
> eine Leitung einer Herzrhythmusbehandlungsvorrichtung;
> eine Leitung einer körpereigenen Herzdefibrillatorvorrichtung;
> eine Führungsvorrichtung einer Leitung einer Herzrhythmusbehandlungsvorrichtung; eine Führungsvorrichtung einer Leitung einer körpereigenen Herzvorrichtung; einen Ventilbehandlungskatheter;
> einen Klappenimplantationskatheter;
> einen körpereigenen Ultraschallkatheter;
> einen körpereigenen Computertomografiekatheter;
> eine therapeutische Nadel;
> eine diagnostische Nadel;
> ein Gastroenterologiegerät;
> eine orthopädische Vorrichtung;
> eine neurochirurgische Vorrichtung;
> eine intravaskuläre Strömungsmessvorrichtung;
> eine intravaskuläre Druckmessvorrichtung;
> eine intravaskuläre optische Kohärenztomografievorrichtung;
> eine intravaskuläre Nah-Infrarot-Spektroskopievorrichtung;
> eine intravaskuläre Infrarotvorrichtung; und
> eine HNO-Präzisionschirurgievorrichtung.

**Revendications**

1. Procédé d'affichage d'une représentation de la pointe d'un dispositif médical situé dans une région de corps d'intérêt (120) du corps d'un patient (122), sur une image de la région de corps d'intérêt, l'image étant acquise par un détecteur d'image (132) d'un imageur mobile (102) à un réglage de zoom physique sélectionné, le procédé comprenant les étapes consistant à :

> acquérir au moins une image de capteur de système de positionnement médical (MPS) d'un capteur MPS, par ledit détecteur d'image (132), à un ou plusieurs réglages de zoom physique dudit détecteur d'image (132) respectif de ladite image, et à un réglage de région de détecteur d'image d'intérêt sélectionné dudit détecteur d'image (132), ledit capteur MPS étant associé à un MPS (104), ledit au moins un capteur MPS (112, 114, 116) répondant à un champ électromagnétique généré par une pluralité de générateurs (118) de champ électromagnétique, qui sont fermement couplés avec une partie mobile dudit imageur mobile (102) ;

déterminer un ensemble de paramètres intrinsèques et extrinsèques au réglage de zoom physique sélectionné, en fonction des coordonnées d'image de capteur de chacune des images de capteur MPS, dans un système de coordonnées optique bidimensionnel respectif dudit détecteur d'image (132), et en fonction des coordonnées MPS en temps non réel dudit capteur MPS (112, 114, 116), dans un système de coordonnées MPS respectif dudit MPS, où chaque ensemble de paramètres intrinsèques comprend des caractéristiques optiques dudit détecteur d'image et chaque ensemble de paramètres extrinsèques comprend des paramètres de rotation et de translation du système de coordonnées MPS par rapport au système de coordonnées optique ;

déterminer des coordonnées bidimensionnelles de ladite pointe dudit dispositif médical, en fonction dudit réglage de zoom physique sélectionné, en fonction dudit ensemble de paramètres intrinsèques et extrinsèques, en fonction dudit réglage de région de détecteur d'image d'intérêt sélectionné, et en fonction des coordonnées MPS en temps réel d'un capteur MPS situé à ladite pointe dudit dispositif médical ;

superposer ladite représentation de ladite pointe dudit dispositif médical, sur ladite image de ladite région de corps d'intérêt, selon lesdites coordonnées bidimensionnelles ; et

afficher ladite représentation de ladite pointe dudit dispositif médical superposée sur ladite image de ladite région de corps d'intérêt, où le procédé est **caractérisé en ce que** ladite étape de détermination dudit ensemble de paramètres intrinsèques et extrinsèques est effectuée par rapport à une pluralité de réglages de zoom physique dudit détecteur d'image, par interpolation entre des paramètres intrinsèques et extrinsèques associés à deux réglages adjacents desdits réglages de zoom physique ou par extrapolation au-delà de paramètres intrinsèques et extrinsèques associés à deux réglages adjacents desdits réglages de zoom physiques.

2. Procédé selon la revendication 1, comprenant en outre une étape consistant à retirer un écran repère à pleine échelle d'un champ de vision dudit détecteur d'image, ledit écran repère à pleine échelle ayant été placé dans ledit champ de vision, dans un mode de fonctionnement hors ligne d'un système fonctionnant selon ledit procédé, ledit écran repère à pleine échelle comprenant une pluralité de repères, comprenant au moins un groupe de repères complémentaires où chaque repère dans un groupe est complémentaire du reste desdits repères dudit groupe.

3. Procédé selon la revendication 2, dans lequel ledit réglage de région de détecteur d'image d'intérêt sélectionné est un réglage parmi un ou plusieurs réglages de région d'intérêt correspondant à une ou plusieurs régions d'intérêt, comprenant en outre une étape consistant à déterminer une fonction d'échelle entre différentes régions de détecteur d'image d'intérêt, en fonction des coordonnées d'image repère dudit au moins un groupe de repères complémentaires dans une pluralité d'images repères acquises par ledit détecteur d'image auxdites différentes régions de détecteur d'image d'intérêt, et audit réglage de zoom physique sélectionné et selon des coordonnées réelles desdits repères complémentaires.

4. Procédé selon la revendication 3, dans lequel ladite procédure de détermination desdites coordonnées est effectuée selon ladite fonction d'échelle.

5. Procédé selon la revendication 3, comprenant en outre une étape consistant à acquérir lesdites images repères par ledit détecteur d'image (132).

6. Procédé selon la revendication 5, comprenant en outre une étape consistant à placer ledit écran repère à pleine échelle dans ledit champ de vision.

7. Procédé selon la revendication 1, dans lequel ladite au moins une image de capteur MPS comprend une image de capteur MPS unique d'une pluralité de capteurs M PS.

8. Procédé selon la revendication 1, dans lequel ladite région de détecteur d'image d'intérêt sélectionné est la plus grande région de détecteur d'image d'intérêt, parmi une pluralité de régions de détecteur d'image d'intérêt.

9. Procédé selon la revendication 1, comprenant en outre une étape consistant à :

fixer fermement un écran de repère périphérique audit détecteur d'image, devant ledit détecteur d'image, dans un mode de fonctionnement en temps non réel d'un système fonctionnant selon ledit procédé, ledit écran de repère périphérique comprenant une pluralité de repères périphériques, comprenant au moins un groupe de repères complémentaires, où chaque repère dans un groupe est complémentaire au reste desdits repères périphériques dans ledit groupe ;

acquérir au moins une image de référence de ladite région de corps d'intérêt, par ledit détecteur d'image dans ledit mode de fonctionnement en temps non réel dudit système, à une position de référence dudit imageur mobile, à chaque réglage de zoom physique dudit détecteur d'image, et à chaque réglage de région de détecteur d'image de d'intérêt dudit détecteur d'image, chacune de ladite au moins une image de référence comprenant une pluralité d'images repères périphériques desdits repères périphériques, à une périphérie de ladite au moins une image de référence ; et

déterminer un modèle de transformation de position de visualisation respectif d'une position de visualisation dudit détecteur d'image, dans un mode de fonctionnement en temps réel dudit système, selon un premier ensemble de coordonnées desdits repères périphériques dans ladite au moins une image de référence, et selon un second ensemble de coordonnées desdits repères périphériques, dans une image en temps réel de ladite région de corps d'intérêt acquise par ledit détecteur d'image.

**10.** Procédé selon la revendication 9, dans lequel ladite étape de détermination desdites coordonnées de ladite pointe dudit dispositif médical, est effectuée en outre selon ledit modèle de transformation de position de visualisation.

**11.** Procédé selon la revendication 9, comprenant en outre les étapes consistant à :

déterminer un ensemble de modèles de correction de rotation d'image respectifs dudit premier ensemble de coordonnées, dans chacune de ladite au moins une image de référence, dans un mode de fonctionnement en temps non réel dudit système ;

construire une relation logique entre chaque modèle de correction de rotation d'image dudit ensemble de modèles de correction de rotation d'image, et ledit premier ensemble respectif de coordonnées, dans ledit mode de fonctionnement en temps non réel dudit système ;

déterminer un modèle de correction de rotation d'image correspondant audit second ensemble de coordonnées, selon ladite relation logique, dans un mode de fonctionnement en temps réel dudit système ; et

effectuer ladite procédure de détermination desdites coordonnées optiques bidimensionnelles de ladite pointe dudit dispositif médical, en outre, selon ledit modèle de correction de rotation d'image.

**12.** Procédé selon la revendication 9, comprenant en outre les étapes consistant à :

déterminer un ensemble de modèles de correction de retournement d'image respectifs dudit premier ensemble de coordonnées, dans chacune de ladite au moins une image de référence, dans un mode de fonctionnement en temps non réel dudit système ;

construire une relation logique entre chaque modèle de correction de retournement d'image dudit ensemble de modèles de correction de rotation d'image, et ledit premier ensemble respectif de coordonnées, dans ledit mode de fonctionnement en temps non réel dudit système ;

déterminer un modèle de correction de retournement d'image correspondant audit second ensemble de coordonnées, selon ladite relation logique, dans un mode de fonctionnement en temps réel dudit système ; et

effectuer ladite procédure de détermination desdites coordonnées optiques bidimensionnelles de ladite pointe dudit dispositif médical, en outre, selon ledit modèle de correction de retournement d'image.

**13.** Procédé selon la revendication 1, comprenant en outre les étapes consistant à :

déterminer une pluralité de modèles de distorsion de position de visualisation correspondant à des valeurs respectives d'une pluralité de valeurs de position de visualisation dudit détecteur d'image, dans un mode de fonctionnement en temps non réel d'un système fonctionnant selon ledit procédé,

construire une première relation logique entre lesdits modèles de distorsion de position de visualisation, et lesdites valeurs de position de visualisation respectives, dans ledit mode de fonctionnement en temps non réel dudit système ;

recevoir des informations respectives d'une valeur de position de visualisation dudit détecteur d'image, à partir d'une interface utilisateur, dans un mode de fonctionnement en temps réel dudit système ; et

déterminer un modèle de distorsion de position de visualisation correspondant à ladite valeur de position de visualisation, en fonction de ladite première relation logique, dans ledit mode de fonctionnement en temps réel dudit système.

**14.** Procédé selon la revendication 13, dans lequel ladite étape de détermination desdites coordonnées bidimensionnelles de ladite pointe dudit dispositif médical, est effectuée en outre selon ledit modèle de transformation de position de visualisation.

**15.** Procédé selon la revendication 13, comprenant en outre les étapes consistant à :

déterminer une pluralité de modèles de correction de rotation d'image correspondant à des valeurs respectives d'une pluralité de valeurs de rotation d'image d'une autre image de ladite région de corps d'intérêt, dans ledit mode de fonctionnement en temps non réel dudit système,

construire une seconde relation logique entre lesdits modèles de correction de rotation d'image et lesdites valeurs de rotation d'image respectives, dans ledit mode de fonctionnement en temps non réel dudit système ;

recevoir des informations relatives à une valeur de rotation d'image de ladite image, à partir d'une interface utilisateur, dans un mode de fonctionnement en temps réel dudit système ; et

déterminer un modèle de correction de rotation d'image correspondant à ladite valeur de rotation d'image, selon ladite seconde relation logique, dans ledit mode de fonctionnement en temps réel dudit système.

**16.** Procédé selon la revendication 13, comprenant en outre les étapes consistant à :

déterminer une pluralité de modèles de correction de retournement d'image correspondant à des valeurs respectives d'une pluralité de valeurs de retournement d'image d'une autre image de ladite région de corps d'intérêt, dans ledit mode de fonctionnement en temps non réel dudit système,

construire une seconde relation logique entre lesdits modèles de correction de retournement d'image et lesdites valeurs de retournement d'image respectives, dans ledit mode de fonctionnement en temps non réel dudit système ;

recevoir des informations respectives d'une valeur de retournement d'image de ladite image, à partir d'une interface utilisateur, dans un mode de fonctionnement en temps réel dudit système ; et

déterminer un modèle de correction de retournement d'image correspondant à ladite valeur de retournement d'image, selon ladite seconde relation logique, dans ledit mode de fonctionnement en temps réel dudit système.

**17.** Procédé selon la revendication 1, comprenant en outre les étapes consistant à :

fixer fermement un écran de repère périphérique audit détecteur d'image, devant ledit détecteur d'image, dans un mode de fonctionnement en temps non réel d'un système fonctionnant selon ledit procédé, ledit écran de repère périphérique comprenant une pluralité de repères périphériques comprenant au moins un groupe de repères périphériques complémentaires, où chaque repère périphérique dans un groupe est complémentaire au reste desdits repères périphériques dans ledit groupe ;

acquérir au moins une image de référence de ladite région de corps d'intérêt, par ledit détecteur d'image dans ledit mode de fonctionnement en temps non réel dudit système, à une position de référence dudit imageur mobile, à chaque réglage de zoom physique dudit détecteur d'image, et à chaque réglage de région de détecteur d'image d'intérêt dudit détecteur d'image, chacune de ladite au moins une image de référence comprenant une pluralité d'images repères périphériques desdits repères périphériques, à une périphérie de ladite au moins une image de référence ;

déterminer une pluralité de modèles de transformation de position de visualisation correspondant à des valeurs respectives d'une pluralité de valeurs de position de visualisation dudit détecteur d'image, dans un mode de fonctionnement en temps non réel d'un système fonctionnant selon ledit procédé, selon des coordonnées d'image repère desdits repères périphériques dans des images respectives de ladite au moins une image de référence, et selon des coordonnées réelles desdits repères périphériques ;

construire une première relation logique entre lesdits modèles de transformation de position de visualisation, et lesdites valeurs de position de visualisation respectives, dans ledit mode de fonctionnement en temps non réel dudit système ;

recevoir des informations respectives d'une valeur de position de visualisation dudit détecteur d'image, provenant dudit détecteur d'image, dans un mode de fonctionnement en temps réel dudit système ; et

déterminer un modèle de transformation de position de visualisation correspondant à ladite valeur de position de visualisation, en fonction de ladite première relation logique, dans ledit mode de fonctionnement en temps réel dudit système.

**18.** Procédé selon la revendication 17, dans lequel ladite étape de détermination desdites coordonnées optiques bidimensionnelles de ladite pointe dudit dispositif médical, est effectuée en outre selon ledit modèle de transformation de position de visualisation.

**19.** Procédé selon la revendication 17, comprenant en outre les étapes consistant à :

déterminer une pluralité de modèles de correction de rotation d'image correspondant à des valeurs respectives d'une pluralité de valeurs de rotation d'image d'une autre image de ladite région de corps d'intérêt, dans ledit mode de fonctionnement en temps non réel dudit système ;
construire une seconde relation logique entre lesdits modèles de correction de rotation d'image et lesdites valeurs de rotation d'image respectives, dans ledit mode de fonctionnement en temps non réel dudit système ;
recevoir des informations respectives d'une valeur de rotation d'image de ladite image, en provenance dudit détecteur d'image, dans un mode de fonctionnement en temps réel dudit système ; et
déterminer un modèle de correction de rotation d'image correspondant à ladite valeur de rotation d'image, selon ladite seconde relation logique, dans ledit mode de fonctionnement en temps réel dudit système.

**20.** Procédé selon la revendication 17, comprenant en outre les étapes consistant à :

déterminer une pluralité de modèles de correction de retournement d'image correspondant à des valeurs respectives d'une pluralité de valeurs de retournement d'image d'une autre image de ladite région de corps d'intérêt, dans ledit mode de fonctionnement en temps non réel dudit système ;
construire une seconde relation logique entre lesdits modèles de correction de retournement d'image et lesdites valeurs de retournement d'image respectives, dans ledit mode de fonctionnement en temps non réel dudit système ;
recevoir des informations respectives d'une valeur de retournement d'image de ladite image, provenant dudit détecteur d'image, dans un mode de fonctionnement en temps réel dudit système ; et
déterminer un modèle de correction de retournement d'image correspondant à ladite valeur de retournement d'image, selon ladite seconde relation logique, dans ledit mode de fonctionnement en temps réel dudit système.

**21.** Procédé selon la revendication 1, comprenant en outre les étapes consistant à :

fixer fermement un écran de repère périphérique audit détecteur d'image, devant ledit détecteur d'image, dans un mode de fonctionnement en temps non réel d'un système fonctionnant selon ledit procédé, ledit écran de repère périphérique comprenant une pluralité de repères périphériques comprenant au moins un groupe de repères complémentaires, où chaque repère périphérique dans un groupe est complémentaire au reste desdits repères périphériques dans ledit groupe ;
acquérir au moins une image de référence de ladite région de corps d'intérêt, par ledit détecteur d'image dans ledit mode de fonctionnement en temps non réel dudit système, à une position de référence dudit imageur mobile, à chaque réglage de zoom physique dudit détecteur d'image, et à chaque région de détecteur d'image d'intérêt dudit détecteur d'image, chacune de ladite au moins une image de référence comprenant une pluralité d'images de repères périphériques desdits repères périphériques, à une périphérie de ladite au moins une image de référence ; et
déterminer un modèle de correction de rotation d'image respectif d'une valeur de rotation d'image de ladite image, dans un mode de fonctionnement en temps réel dudit système, selon un premier ensemble de coordonnées desdits repères périphériques dans ladite au moins une image de référence, et selon un second ensemble de coordonnées desdits repères périphériques, dans ladite image.

**22.** Procédé selon la revendication 21, dans lequel ladite étape de détermination desdites coordonnées de ladite pointe dudit dispositif médical, est effectuée en outre selon ledit modèle de correction de rotation d'image.

**23.** Procédé selon la revendication 1, comprenant en outre les étapes consistant à :

fixer fermement un écran de repère périphérique audit détecteur d'image, devant ledit détecteur d'image, dans un mode de fonctionnement en temps non réel d'un système fonctionnant selon ledit procédé, ledit écran de repère périphérique comprenant une pluralité de repères périphériques comprenant au moins un groupe de repères complémentaires, où chaque repère périphérique dans un groupe est complémentaire au reste desdits repères périphériques dans ledit groupe ;
acquérir au moins une image de référence de ladite région de corps d'intérêt, par ledit détecteur d'image dans ledit mode de fonctionnement en temps non réel dudit système, à une position de référence dudit imageur mobile, à chaque réglage de zoom physique dudit détecteur d'image, et à chaque région de détecteur d'image d'intérêt dudit détecteur d'image, chacune de ladite au moins une image de référence comprenant une pluralité d'images repères périphériques desdits repères périphériques, à une périphérie de ladite au moins une image de référence ; et
déterminer un modèle de correction de retournement d'image respectif d'une valeur de rotation d'image de ladite image, dans un mode de fonctionnement en temps réel dudit système, selon un premier ensemble de coordonnées desdits repères périphériques dans ladite au moins une image de référence, et selon un second ensemble de coordonnées desdits repères périphériques, dans ladite image.

24. Procédé selon la revendication 23, dans lequel ladite étape de détermination desdites coordonnées de ladite pointe dudit dispositif médical, est effectuée en outre selon ledit modèle de correction de retournement d'image.

25. Procédé selon la revendication 1, comprenant en outre les étapes consistant à :

déterminer une pluralité de modèles de correction de rotation d'image correspondant à des valeurs respectives d'une pluralité de valeurs de rotation d'image d'une autre image de ladite région de corps d'intérêt, dans ledit mode de fonctionnement en temps non réel dudit système ;
construire une relation logique entre lesdits modèles de correction de rotation d'image et lesdites valeurs de rotation d'image respectives, dans ledit mode de fonctionnement en temps non réel dudit système ;
recevoir des informations relatives à une valeur de rotation d'image de ladite image, à partir d'une interface utilisateur, dans un mode de fonctionnement en temps réel dudit système ; et
déterminer un modèle de correction de rotation d'image correspondant à ladite valeur de rotation d'image, selon ladite relation logique, dans ledit mode de fonctionnement en temps réel dudit système.

26. Procédé selon la revendication 1, comprenant en outre les étapes consistant à :

déterminer une pluralité de modèles de correction de retournement d'image correspondant à des valeurs respectives d'une pluralité de valeurs de retournement d'image d'une autre image de ladite région de corps d'intérêt, dans ledit mode de fonctionnement en temps non réel dudit système ;
construire une relation logique entre lesdits modèles de correction de retournement d'image et lesdites valeurs de retournement d'image respectives, dans ledit mode de fonctionnement en temps non réel dudit système ;
recevoir des informations respectives d'une valeur de retournement d'image de ladite image, à partir d'une interface utilisateur, dans un mode de fonctionnement en temps réel dudit système ; et
déterminer un modèle de correction de retournement d'image correspondant à ladite valeur de retournement d'image, selon ladite relation logique, dans ledit mode de fonctionnement en temps réel dudit système.

27. Procédé selon la revendication 1, comprenant en outre les étapes consistant à :

déterminer une pluralité de modèles de correction de rotation d'image correspondant à des valeurs respectives d'une pluralité de valeurs de rotation d'image d'une autre image de ladite région de corps d'intérêt, dans ledit mode de fonctionnement en temps non réel dudit système ;
construire une relation logique entre lesdits modèles de correction de rotation d'image et lesdites valeurs de rotation d'image respectives, dans ledit mode de fonctionnement en temps non réel dudit système ;
recevoir des informations respectives d'une valeur de rotation d'image de ladite image, en provenance dudit détecteur d'image, dans un mode de fonctionnement en temps réel dudit système ; et
déterminer un modèle de correction de rotation d'image correspondant à ladite valeur de rotation d'image, selon ladite relation logique, dans ledit mode de fonctionnement en temps réel dudit système.

28. Procédé selon la revendication 1, comprenant en outre les étapes consistant à :

déterminer une pluralité de modèles de correction de retournement d'image correspondant à des valeurs respectives d'une pluralité de valeurs de retournement d'image d'une autre image de ladite région de corps d'intérêt, dans ledit mode de fonctionnement en temps non réel dudit système ;
construire une relation logique entre lesdits modèles de correction de retournement d'image et lesdites valeurs de retournement d'image respectives, dans ledit mode de fonctionnement en temps non réel dudit système ;
recevoir des informations respectives d'une valeur de retournement d'image de ladite image, provenant dudit détecteur d'image, dans un mode de fonctionnement en temps réel dudit système ; et
déterminer un modèle de correction de retournement d'image correspondant à ladite valeur de retournement d'image, selon ladite relation logique, dans ledit mode de fonctionnement en temps réel dudit système.

29. Procédé selon la revendication 1, dans lequel ladite représentation et ladite image sont chacune en temps réel.

**30.** Procédé selon la revendication 1, dans lequel ladite représentation est en temps réel et ladite image est acquise précédemment.

**31.** Procédé selon la revendication 1, dans lequel ladite représentation est acquise précédemment et ladite image est en temps réel.

**32.** Procédé selon la revendication 1, dans lequel ladite représentation et ladite image sont chacune acquise précédemment.

**33.** Système d'affichage d'une représentation de la pointe d'un dispositif médical situé dans une région de corps d'intérêt (120) d'un patient (122), sur une image de la région de corps d'intérêt, l'image étant acquise par un détecteur d'image (132) d'un imageur mobile (102) à un ou plusieurs réglages de zoom physique dudit détecteur d'image (132) respectif de ladite image, et à un réglage de région de détecteur d'image d'intérêt sélectionné dudit détecteur d'image (132), le système comprenant :

au moins un générateur de champ magnétique (118) couplé fermement à une partie mobile dudit imageur mobile (102), ledit au moins un générateur de champ magnétique (118) produisant un champ magnétique au niveau de ladite région de corps d'intérêt ;

un capteur (112, 114, 116) de système de positionnement médical (MPS) de dispositif médical couplé à ladite pointe dudit dispositif médical, ledit capteur MPS de dispositif médical (112, 114, 116) détectant ledit champ magnétique ;

un MPS (104) couplé audit au moins un générateur de champ magnétique (118) et audit capteur MPS de dispositif médical (112, 114, 116), ledit au moins un générateur de champ magnétique (118) étant associé à un système de coordonnées MPS respectif dudit MPS (102), ledit MPS déterminant les coordonnées MPS dudit capteur MPS de dispositif médical (112, 114, 116), selon une sortie dudit capteur MPS de dispositif médical (112, 114, 116) ; et

un processeur (108) couplé audit MPS (102), ledit processeur (108) déterminant des coordonnées bidimensionnelles de ladite pointe dudit dispositif médical situé dans ladite région de corps d'intérêt, selon un réglage de zoom physique dudit détecteur d'image (132) respectif de ladite image, selon un ensemble de paramètres intrinsèques et extrinsèques respectifs dudit détecteur d'image (132), ledit ensemble de paramètres intrinsèques et extrinsèques étant déterminé au niveau d'un réglage de zoom physique sélectionné, selon des coordonnées d'image de capteur de chacune des images de capteur MPS dans un système de coordonnées optique bidimensionnel respectif dudit détecteur d'image (132) et selon les coordonnées MPS en temps non réel dudit capteur MPS, dans un système de coordonnées MPS respectif dudit MPS, dans lequel chaque ensemble de paramètres intrinsèques comprenant des caractéristiques optiques dudit détecteur d'image et chaque ensemble de paramètres extrinsèques comprenant des paramètres de rotation et de translation du système de coordonnées MPS relativement au système de coordonnées bidimensionnelles, selon ledit réglage de région de détecteur d'image d'intérêt sélectionné dudit détecteur d'image, et selon lesdites coordonnées MPS dudit capteur MPS de dispositif médical, ledit processeur superposant une représentation de ladite pointe dudit dispositif médical, sur ladite image, selon lesdites coordonnées bidimensionnelles, où le processeur (108) est **caractérisé en ce qu'**il est adapté pour déterminer ledit ensemble de paramètres intrinsèques et extrinsèques par rapport à une pluralité de réglages de zoom physique dudit détecteur d'image, en interpolant entre des paramètres intrinsèques et extrinsèques associés à deux réglages adjacents parmi lesdits réglages de zoom physique ou en extrapolant au-delà des paramètres intrinsèques et extrinsèques associés à deux réglages adjacents parmi lesdits réglages de zoom physique.

**34.** Système selon la revendication 33, comprenant en outre une interface utilisateur couplée audit processeur, ladite interface utilisateur recevant une entrée d'un utilisateur.

**35.** Système selon la revendication 34, dans lequel ladite entrée est sélectionnée dans la liste constituée par :

un angle de rotation de ladite image ;
un type de retournement de ladite image ; et
une valeur de position de visualisation dudit détecteur d'image.

**36.** Système selon la revendication 33, comprenant en outre un affichage couplé audit processeur, ledit affichage affichant une superposition de ladite représentation sur ladite image.

37. Système selon la revendication 33, dans lequel ledit réglage de région de détecteur d'image d'intérêt sélectionné est un réglage parmi un ou plusieurs réglages de région de détecteur d'image d'intérêt correspondant à une ou plusieurs régions d'intérêt, le système comprenant en outre un écran repère pleine échelle couplé audit détecteur d'image, devant ledit détecteur d'image, dans un mode de fonctionnement hors ligne dudit système, ledit écran repère pleine échelle comprenant une pluralité de repères comprenant au moins un groupe de repères complémentaires, où chaque repère dans un groupe est complémentaire au reste desdits repères dans ledit groupe, ledit processeur déterminant une fonction d'échelle entre différentes régions de détecteur d'image d'intérêt, en fonction des coordonnées d'image repères desdits repères, dans une pluralité d'images repères acquises par ledit détecteur d'image, à partir dudit écran repères à pleine portée, au niveau desdites différentes régions de détecteur d'image d'intérêt, et au niveau d'au moins un réglage de zoom physique dudit détecteur d'image, et selon des coordonnées réelles desdits repères.

38. Système selon la revendication 33, comprenant en outre un écran repère périphérique situé dans un champ de vision dudit détecteur d'image, dans un mode de fonctionnement en temps non réel dudit système, ledit écran repère périphérique comprenant une pluralité de repères périphériques comprenant au moins un groupe de repères complémentaires, dans lequel chaque repère périphérique dans un groupe est complémentaire au reste desdits repères périphériques dans ledit groupe, ledit détecteur d'image acquérant au moins une image de référence de ladite région de corps d'intérêt, dans ledit mode de fonctionnement en temps non réel dudit système, à une position de référence dudit imageur mobile, à chaque réglage de zoom physique dudit détecteur d'image, et à chaque réglage de région de détecteur d'image d'intérêt dudit détecteur d'image, chacune de ladite au moins une image de référence comprenant une pluralité d'images repères périphériques desdits repères périphériques, à une périphérie de ladite au moins une image de référence,
dans lequel ledit processeur détermine un modèle de transformation de position de visualisation respectif d'une position de visualisation sélectionnée dudit détecteur d'image, dans un mode de fonctionnement en temps réel dudit système, selon un premier ensemble de coordonnées desdits repères périphériques dans ladite au moins une image de référence, et selon un second ensemble de coordonnées desdits repères périphériques, dans ladite image.

39. Système selon la revendication 38, comprenant en outre une base de données couplée audit processeur, dans lequel ledit processeur détermine une pluralité de modèles de correction de rotation d'image, respectifs des valeurs respectives d'une pluralité de valeurs de rotation d'image de ladite au moins une image de référence, selon ledit premier ensemble de coordonnées, dans ledit mode de fonctionnement non temps réel dudit système,
dans lequel ledit processeur construit une relation logique entre lesdits modèles de correction de rotation d'image, et lesdites valeurs de rotation d'image, dans ledit mode de fonctionnement en temps non réel dudit système,
dans lequel ledit processeur stocke ladite relation logique dans ladite base de données, dans lequel ledit processeur détermine un modèle de correction de rotation d'image correspondant à une valeur de rotation d'image sélectionnée de ladite image, dans ledit mode de fonctionnement en temps réel dudit système, en incorporant ledit second ensemble de coordonnées dans ladite relation logique, et
dans lequel ledit processeur détermine lesdites coordonnées de ladite pointe dudit dispositif médical, en outre selon ledit modèle de correction de rotation d'image.

40. Système selon la revendication 38, comprenant en outre une base de données couplée audit processeur, dans lequel ledit processeur détermine une pluralité de modèles de correction de retournement d'image, respectifs de valeurs respectives d'une pluralité de valeurs de retournement d'image de ladite au moins une image de référence, selon ledit premier ensemble de coordonnées, dans ledit mode de fonctionnement en temps non réel dudit système,
dans lequel ledit processeur construit une relation logique entre lesdits modèles de correction de retournement d'image, et lesdites valeurs de retournement d'image, dans ledit mode de fonctionnement en temps non réel dudit système,
dans lequel ledit processeur stocke ladite relation logique dans ladite base de données,
dans lequel ledit processeur détermine un modèle de correction de retournement d'image correspondant à une valeur de retournement d'image sélectionnée de ladite image, dans ledit mode de fonctionnement en temps réel dudit système, en incorporant ledit second ensemble de coordonnées dans ladite relation logique, et
dans lequel ledit processeur détermine lesdites coordonnées de ladite pointe dudit dispositif médical, en outre selon ledit modèle de correction de retournement d'image.

41. Système selon la revendication 33, comprenant en outre :

un écran de repère périphérique situé dans un champ de vision dudit détecteur d'image, dans un mode de fonctionnement en temps non réel dudit système, ledit écran de repère périphérique comprenant une pluralité de repères périphériques comprenant au moins un groupe de repères complémentaires, dans lequel chaque repère périphérique dans un groupe est complémentaire au reste desdits repères périphériques dans ledit groupe, ledit détecteur d'image acquérant au moins une image de référence de ladite région de corps d'intérêt, dans ledit mode de fonctionnement en temps non réel dudit système, à une position de référence dudit imageur mobile, à chaque réglage de zoom physique dudit détecteur d'image, et à chaque réglage de région de détecteur d'image d'intérêt dudit détecteur d'image, chacune de ladite au moins une image de référence comprenant une pluralité d'images repères périphériques desdits repères périphériques, à une périphérie de ladite au moins une image de référence ;

une base de données couplée audit processeur, où ledit processeur détermine une pluralité de modèles de transformation de position de visualisation correspondant à des valeurs respectives d'une pluralité de valeurs de position de visualisation dudit détecteur d'image, dans un mode de fonctionnement en temps non réel dudit système, selon des coordonnées d'image repères desdits repères périphériques dans les images respectives de ladite au moins une image de référence, et selon les coordonnées réelles desdits repères périphériques ;

dans lequel ledit processeur construit une première relation logique entre lesdits modèles de transformation de position de visualisation, et lesdites valeurs de position de visualisation respectives, dans ledit mode de fonctionnement en temps non réel dudit système,

dans lequel ledit processeur reçoit des informations respectives d'une valeur de position de visualisation dudit détecteur d'image, depuis une interface utilisateur, dans un mode de fonctionnement en temps réel dudit système ; et

dans lequel ledit processeur détermine un modèle de transformation de position de visualisation correspondant à ladite valeur de position de visualisation, selon ladite première relation logique, dans ledit mode de fonctionnement en temps réel dudit système.

**42.** Système selon la revendication 33, comprenant en outre un détecteur de position couplé audit imageur mobile et audit processeur, ledit processeur déterminant une position dudit imageur mobile en fonction d'une sortie dudit détecteur de position.

**43.** Système selon la revendication 33, comprenant en outre un capteur MPS de référence fixé à un emplacement de référence, ledit capteur MPS de référence étant couplé audit MPS, ledit MPS déterminant une position dudit imageur mobile selon une sortie dudit capteur MPS de référence.

**44.** Système selon la revendication 33, comprenant en outre un capteur MPS de détecteur d'image couplé audit détecteur d'image et audit MPS, ledit imageur mobile comprenant un émetteur situé sur un côté opposé dudit patient par rapport à l'emplacement dudit détecteur d'image, ledit émetteur émettant un rayonnement vers ledit détecteur d'image suivant un axe de rayonnement, ledit MPS déterminant une position dudit détecteur d'image suivant ledit axe de rayonnement, selon une sortie dudit capteur MPS de détecteur d'image.

**45.** Système selon la revendication 33, comprenant en outre un capteur MPS de corps de patient couplé fermement avec le corps dudit patient et avec ledit MPS, ledit MPS déterminant une valeur de position de visualisation dudit détecteur d'image par rapport audit corps, selon une sortie dudit capteur MPS de corps du patient, ledit processeur compensant les mouvements dudit patient et dudit imageur mobile, tandis que ledit processeur traite des données respectives des images que ledit détecteur d'image détecte.

**46.** Système selon la revendication 33, dans lequel chacune de ladite représentation et de ladite image est en temps réel.

**47.** Système selon la revendication 33, dans lequel ladite représentation est en temps réel et ladite image est acquise précédemment.

**48.** Système selon la revendication 33, dans lequel ladite représentation est acquise précédemment et ladite image est en temps réel.

**49.** Système selon la revendication 33, dans lequel chacune de ladite représentation et de ladite image est acquise précédemment.

**50.** Système selon la revendication 33, dans lequel ledit détecteur d'image est un intensificateur d'image.

**51.** Système selon la revendication 33, dans lequel ledit détecteur d'image est un détecteur plat.

**52.** Système selon la revendication 33, dans lequel lesdits générateurs de champ magnétique sont couplés audit détecteur d'image.

**53.** Système selon la revendication 33, dans lequel ledit imageur mobile comprend un émetteur situé sur un côté opposé dudit patient par rapport à l'emplacement dudit détecteur d'image, ledit émetteur émettant un rayonnement vers ledit détecteur d'image, lesdits générateurs de champ magnétique étant couplés avec ledit émetteur.

**54.** Système selon la revendication 33, dans lequel ledit imageur mobile est une machine de tomographie assistée par ordinateur (TAO), ledit TAO comprenant un détecteur d'image TAO et un émetteur TAO, ledit émetteur TAO étant situé sur un côté opposé dudit patient par rapport à l'emplacement dudit détecteur d'image TAO, ledit émetteur TAO émettant un rayonnement vers ledit détecteur d'image TAO, lesdits générateur de champ magnétique étant couplés avec ledit détecteur d'image TAO.

**55.** Système selon la revendication 33, dans lequel ledit imageur mobile fonctionne selon un principe sélectionné dans la liste constituée de :

radios ;
résonance magnétique nucléaire ;
émission de particules élémentaires ;
et thermographie.

**56.** Système selon la revendication 33, dans lequel ledit dispositif médical est sélectionné parmi la liste constituée de :

cathéter à ballonnet ;
cathéter porteur d'endoprothèse ;
cathéter de distribution de substances médicales ;
cathéter de suture ;
fil-guide ;
unité d'ablation ;
unité de curiethérapie ;
cathéter d'échographie intravasculaire ;
sonde d'un dispositif de traitement de rythme cardiaque ;
sonde d'un dispositif défibrillateur cardiaque intracorporel ;
dispositif de guidage d'une sonde d'un dispositif de traitement du rythme cardiaque ; dispositif de guidage d'une sonde d'un dispositif cardiaque intracorporel ; cathéter de traitement valvulaire ;
cathéter d'implantation valvulaire ;
cathéter d'échographie intracorporel ;
cathéter de tomodensitométrie intracorporel ;
aiguille thérapeutique ;
aiguille de diagnostic ;
dispositif gastro-entérologie ;
dispositif orthopédique ;
dispositif neurochirurgical ;
dispositif de mesure de débit intravasculaire ;
dispositif de mesure de pression intravasculaire ;
dispositif de tomographie à cohérence optique intravasculaire ;
dispositif de spectroscopie intravasculaire dans l'infrarouge proche ;
dispositif à infrarouge intravasculaire ; et
dispositif de chirurgie de précision en oto-rhino-laryngologie.

FIG. 1
PRIOR ART

EP 1 944 733 B1

FIG. 2

ACQUIRING AT LEAST ONE MPS SENSOR IMAGE OF AT LEAST ONE MPS SENSOR, BY AN IMAGE DETECTOR OF A MOVING IMAGER, AT A PHYSICAL ZOOM SETTING OF THE IMAGE DETECTOR, RESPECTIVE OF AN IMAGE OF A BODY REGION OF INTEREST OF THE BODY OF A PATIENT, AND AT A SELECTED IMAGE DETECTOR REGION OF INTEREST SETTING OF THE IMAGE DETECTOR, THE MPS SENSOR BEING ASSOCIATED WITH AN MPS, THE MPS SENSOR RESPONDING TO AN ELECTROMAGNETIC FIELD GENERATED BY A PLURALITY OF ELECTROMAGNETIC FIELD GENERATORS, FIRMLY COUPLED WITH A MOVING PORTION OF THE MOVING IMAGER
160

DETERMINING A SET OF INTRINSIC AND EXTRINSIC PARAMETERS, ACCORDING TO SENSOR IMAGE COORDINATES OF EACH OF THE MPS SENSOR IMAGES, IN A 2D OPTICAL COORDINATE SYSTEM RESPECTIVE OF THE IMAGE DETECTOR, AND ACCORDING TO THE RESPECTIVE MPS COORDINATES OF THE MPS SENSOR, IN AN MPS COORDINATE SYSTEM RESPECTIVE OF THE MPS
162

DETERMINING 2D OPTICAL COORDINATES OF THE TIP OF A CATHETER LOCATED WITHIN THE BODY REGION OF INTEREST, ACCORDING TO THE PHYSICAL ZOOM SETTING, ACCORDING TO THE SET OF INTRINSIC AND EXTRINSIC PARAMETERS, ACCORDING TO THE SELECTED IMAGE DETECTOR REGION OF INTEREST SETTING, AND ACCORDING TO MPS COORDINATES OF THE MPS SENSOR ATTACHED TO THE TIP OF THE CATHETER
164

SUPERIMPOSING A REPRESENTATION OF THE TIP OF THE CATHETER, ON AN IMAGE OF THE BODY REGION OF INTEREST, ACCORDING TO THE DETERMINED 2D OPTICAL COORDINATES
166

FIG. 3

EP 1 944 733 B1

FIG. 4

EP 1 944 733 B1

FIG. 5

EP 1 944 733 B1

**EP 1 944 733 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2006058647 A1
- US 6203493 B1
- US 6366799 B1, Acker